# EUROPEAN PATENT APPLICATION

(11) **EP 3 187 114 A1**
(43) Date of publication of application: **05.07.2017**
(21) Application number: 15835014.0
(22) Date of filing: 21.08.2015
(51) Int. Cl.: A61B 8/12, A61B 8/13

(54) **PHOTOACOUSTIC WAVE DETECTOR AND PHOTOACOUSTIC IMAGING DEVICE**

(30) Priority: 28.08.2014 JP 2014173552; 28.08.2014 JP 2014174290
(71) Applicant: PreXion Corporation, Chiyoda-ku Tokyo 101-0041 (JP)
(72) Inventor: SHIGETA, Yusuke, Tokyo 101-0041 (JP)
(74) Representative: Grünecker Patent- und Rechtsanwälte PartG mbB
(86) International application number: PCT/JP2015/073495
(87) International publication number: WO 2016/031700

(57) **Abstract**

This photoacoustic wave detector is provided with: an internal light source (212) that comprises an LED element (212a) to be inserted into the interior of a subject (P) and that irradiates an object to be detected (Q) within the subject with light emitted from the LED element; and an internal acoustoelectric conversion unit (22) that detects a photoacoustic wave generated at the object to be detected in the interior of the subject (150) and generates an internal detection signal that is based on the detection result of the photoacoustic wave (AW).

## Description

### Technical Field

The present invention relates to a photoacoustic wave detector and a photoacoustic imaging apparatus. The present invention specifically relates to a photoacoustic wave detector including a detecting portion for detecting a photoacoustic wave generated from a detection target when the detection target absorbs light emitted from a light source, and a photoacoustic imaging apparatus.

### Background Art

A photoacoustic wave detector conventionally known includes a detecting portion for detecting a photoacoustic wave generated from a detection target when the detection target absorbs light emitted from a light source. Such a photoacoustic wave detector is disclosed in Japanese Patent Laying-Open No. 2012-249739, for example.

The photoacoustic wave detector disclosed in Japanese Patent Laying-Open No. 2012-249739 includes an ultrasonic endoscope capable of increasing an intensity in detecting a photoacoustic wave generated at a detection target existing at a deep area inside a test object. This photoacoustic wave detector includes an optical fiber, a balloon having a light scattering structure, and a vibrator array that are provided at the tip of the ultrasonic endoscope. The optical fiber is configured to guide a laser beam generated outside the ultrasonic endoscope to the tip of the ultrasonic endoscope inserted into the test object. At the tip of the ultrasonic endoscope, the laser beam emitted from the optical fiber and scattered by the balloon is applied to the inside of the test object. The photoacoustic wave detector is configured to acquire a tomographic image of the deep area inside the test object by making the vibrator array at the tip of the ultrasonic endoscope detect a photoacoustic wave generated at the detection target when the detection target absorbs the laser beam.

### Citation List

### Patent Literature

Patent Literature 1: Japanese Patent Laying-Open No. 2012-249739

### Summary of Invention

### Technical Problem

In the above-described photoacoustic endoscope described in Japanese Patent Laying-Open No. 2012-249739, however, using a laser beam of high light output requires provision of a device for generating a laser beam to a photoacoustic imaging apparatus. Additionally, a light guide member such as the optical fiber and a scattering member are also required to be provided to the ultrasonic endoscope. Hence, size increase and complication of the apparatus become unavoidable and cost reduction of the apparatus is difficult to achieve. Meanwhile, if light is generated outside the ultrasonic endoscope by using a light-emitting element, etc. for emitting light of lower output than a laser beam, the light generated outside the ultrasonic endoscope cannot be guided easily to the ultrasonic endoscope as a result of a larger distribution angle of light emitted from the light-emitting element than a laser beam. Guiding light from a low-output light source to the ultrasonic endoscope might achieve size reduction of the apparatus. However, this is considered to be a cause that makes it difficult to detect a photoacoustic wave of an intensity sufficient for generating a tomographic image of the deep area inside the test object.

The present invention has been made to solve the above-described problem. It is one object of the present invention to provide a photoacoustic wave detector and a photoacoustic imaging apparatus capable of detecting a photoacoustic wave of an intensity sufficient for generating a tomographic image of a deep area inside a test object by using a relatively low-output light source.

### Solution to Problem

To attain the aforementioned object, a photoacoustic wave detector according to a first aspect of the present invention includes: an internal light source including a light-emitting element to be inserted into a test object, the internal light source applying light emitted from the light-emitting element to a detection target inside the test object; and an internal detecting portion that detects a photoacoustic wave inside the test object and generates an internal detection signal based on a result of detection of the photoacoustic wave, the photoacoustic wave being generated at the detection target.

In this way, light is emitted from the light-emitting element in the test object and the photoacoustic wave is detected also in the test object. Thus, even if the detection target is at a deep area inside the test object, light can be emitted and the photoacoustic wave can be detected at a position relatively close to the detection target. This makes it possible to detect the photoacoustic wave of an intensity sufficient for generating a tomographic image of the deep area inside the test object by using a relatively low-output light source.

In the photoacoustic wave detector according to the aforementioned first aspect, the internal light source and the internal detecting portion are both preferably provided near the tip of an inserted part to be inserted into the test object. In this configuration, the internal light source and the internal detecting portion are provided near the tip of the inserted part, so that a photoacoustic image of a deeper area inside the test object can be acquired.

In the photoacoustic wave detector according to the aforementioned first aspect, the internal detecting portion is preferably provided at a lateral surface of the inserted part to be inserted into the test object, the lateral surface extending in a direction conforming to a direction of insertion of the inserted part. Further, the lateral surface is preferably formed of a curved surface or a plurality of planes and includes a mounting surface on which the light-emitting element is mounted, and the light-emitting element is preferably arranged to be pointed outward of a direction of a normal to the mounting surface of the inserted part. This configuration makes it possible to generate a tomographic image taken along the lateral surface of the inserted part of the photoacoustic wave detector to be inserted into the test object.

In this case, the mounting surface is preferably formed of a circular curved surface, the internal detecting portion is preferably provided along the outer periphery of the lateral surface of the inserted part as viewed in the direction of insertion of the inserted part, and the light-emitting element is preferably configured to emit light outward of the direction of the normal so as to cover the entire periphery of the mounting surface as viewed in the direction of insertion of the inserted part. This configuration makes it possible to generate a tomographic image covering a wide range along the entire periphery of the lateral surface of the inserted part of the photoacoustic wave generator.

In the aforementioned configuration where the light-emitting element is pointed outward of the direction of the normal to the mounting surface of the inserted part, the photoacoustic wave detector preferably further includes a reflective member that reflects light emitted from the light-emitting element outward of the direction of the normal. In this configuration, emitted light to travel in a direction toward the direction of insertion is reflected on the reflective surface to a direction toward the direction of the normal to the lateral surface of the inserted part. This achieves efficient emission of light to the test object.

The photoacoustic wave detector according to the aforementioned first aspect preferably further includes a reflective member that reflects light emitted from the light-emitting element outward of the direction of the normal to the lateral surface of the inserted part to be inserted into the test object, the lateral surface extending in a direction conforming to the direction of insertion of the inserted part. Further, the internal detecting portion is preferably provided at the lateral surface, the light-emitting element is preferably mounted on a mounting surface pointed in the direction of insertion, and the reflective member preferably includes a reflective surface facing the mounting surface. In this configuration, emitted light to travel in a direction toward the direction of insertion can be reflected on the reflective surface and the reflected light can be reflected to a direction toward a direction of a normal to an outer peripheral surface of the internal detecting portion. This achieves efficient emission of light to the test object. This further makes it possible to emit light easily to a region in the vicinity of the internal detecting portion. Thus, even if the detection target is near the internal detecting portion, the photoacoustic wave of a sufficient intensity can still be detected.

In the photoacoustic wave detector according to the aforementioned first aspect, the inserted part to be inserted into the test object is preferably rotatable about the direction of insertion of the inserted part as an axis. This makes it possible to change a direction of application of light to any direction perpendicular to the direction of insertion. As a result, a measurement region to appear in a tomographic image can be changed arbitrarily.

In the photoacoustic wave detector according to the aforementioned first aspect, the internal light source preferably includes at least one of a light-emitting diode element, a semiconductor laser element, and an organic light-emitting diode element to function as the light-emitting element. This configuration achieves a relatively large angle of distribution of light from the light-emitting element, so that light can be emitted uniformly in the direction of the normal to the lateral surface of the inserted part. This configuration further achieves size reduction of the internal light source. Additionally, pulsed light of a high light emission frequency can be emitted to the test object using a simple configuration. This makes it possible to acquire more tomographic images per unit time. As a result, a photoacoustic image having a higher degree of clearness and higher resolution can be acquired.

In the photoacoustic wave detector according to the aforementioned first aspect, the internal detecting portion preferably includes a detection surface provided near the internal light source and used for detecting a photoacoustic wave. The photoacoustic wave detector preferably further includes a reflective portion having a reflective surface on which light generated from the light-emitting element is reflected toward a direction of the detection target facing the detection surface. This configuration allows light generated from the light-emitting element of the endoscope body to be emitted directly to the test object. This eliminates the need for a configuration conventionally required for guiding light from a light source provided separately from the endoscope body to the endoscope body, thereby contributing to simplification of the configuration of a light transmission path. Further, unlike in a conventional case of using a solid-state laser, using the light-emitting element such as a light-emitting diode element can contribute to size reduction of the configuration of a light source. Further, as a result of provision of the reflective portion having the reflective surface to the endoscope body, emitted light not to travel in the direction of the detection target can be reflected on the reflective surface toward the detection target. This allows efficient emission of light to the detection target.

In the aforementioned configuration further including the reflective portion, the reflective portion preferably includes a side wall arranged lateral to the internal detecting portion and the internal light source, and the reflective surface is preferably provided on a surface of the side wall adjacent to the internal detecting portion and the internal light source. In this configuration, even if a light-emitting diode likely to cause diffusion of light is used as the light-emitting element, for example, light can be emitted to the detection target in a well-balanced manner only by providing the reflective surface as a simple structure. Specifically, well-balanced emission of light to the detection target is achieved without complicating the configuration of a light transmission path.

The aforementioned configuration further including the reflective portion preferably further includes a light guide portion that guides light from the internal light source to the test object. The internal light source preferably includes a sealing portion that seals the light-emitting element, and the light guide portion is preferably arranged closer to the detection target than the sealing portion. This configuration makes it possible to guide light from the sealing portion to the test object through the light guide portion, thereby suppressing loss of light energy occurring between the sealing portion and the test object.

In the aforementioned configuration further including the light guide portion, the sealing portion preferably has an outer surface formed into a lens-like shape that collects light from the light-emitting element and directs the collected light toward the light guide portion. In this configuration, light from the light-emitting element can be collected and directed to the light guide portion, thereby suppressing loss of light energy occurring from emission of light from the light-emitting element to arrival of the light at the light guide portion.

In the aforementioned configuration further including the light guide portion, the internal detecting portion preferably includes a piezoelectric element that allows transmission of light, and the piezoelectric element is preferably arranged in the light guide portion and is arranged to overlap the light-emitting element in a plan view. In this configuration, the light-emitting element is not required to be arranged so as to bypass the position of the piezoelectric element in a plan view. This can ensure a wider area for arrangement of the light-emitting element than arrangement where the light-emitting element and the piezoelectric element do not overlap in a plan view. As a result, the quantity of light from the light source can be increased.

In the aforementioned configuration further including the reflective portion, the internal light source preferably includes a plurality of the light-emitting elements, and the reflective surface is preferably provided to surround the light-emitting elements. In this configuration, as a result of the presence of the reflective surface surrounding the light-emitting elements, light emitted from the light-emitting element to be diffused to its surrounding can be reflected toward the detection target. As a result, the light can be emitted efficiently to the detection target.

In the aforementioned configuration where the reflective surface surrounds the light-emitting elements, the reflective surface is preferably provided in an annular shape to surround the entire peripheries of the light-emitting elements. This configuration allows the reflective surface to cause reflection of light over the entire peripheries of the light-emitting elements, so that the light can be emitted more efficiently to the detection target.

A photoacoustic imaging apparatus according to a second aspect of the present invention includes: a photoacoustic wave detector including an internal light source and an internal detecting portion, the internal light source including a light-emitting element to be inserted into a test object, the internal light source applying light emitted from the light-emitting element to a detection target inside the test object, the internal detecting portion detecting a photoacoustic wave in the test object and generating an internal detection signal based on a result of detection of the photoacoustic wave, the photoacoustic wave being generated at the detection target; an internal detection image generating portion that generates an internal detection tomographic image of the test object based on the internal detection signal; and an image forming portion that forms a photoacoustic image using the internal detection tomographic image.

In this way, emission of light from the light-emitting element and detection of a photoacoustic wave can be achieved inside the test object. Further, the internal detection tomographic image is generated based on the internal detection signal about the photoacoustic wave generated inside the test object and the photoacoustic image is formed using the internal detection tomographic image. Thus, even if the detection target is at a deep area inside the test object, light can still be emitted and the photoacoustic wave can still be detected at a position relatively close to the detection target. Further, a photoacoustic image of the detection target can be formed. This makes it possible to detect the photoacoustic wave of an intensity sufficient for generating a tomographic image of the deep area inside the test object and to form a photoacoustic image of the detected photoacoustic wave by using a relatively low-output light source.

The photoacoustic imaging apparatus according to the aforementioned second aspect preferably further includes an external light source that emits light from the outside of the test object to the detection target. Timing of emission of light from the external light source is preferably synchronized with timing of emission of light from the internal light source. In this configuration, the external light source at a surface of the test object becomes usable as an auxiliary light source, so that an intensity in detecting the photoacoustic wave can be increased further.

The photoacoustic imaging apparatus according to the aforementioned second aspect preferably further includes: an external detecting portion that detects a photoacoustic wave outside the test object and generates an external detection signal based on a result of detection of the photoacoustic wave, the photoacoustic wave being generated at the detection target in the test object; and an external detection image generating portion that generates an external detection tomographic image of the test object based on the external detection signal. The image forming portion preferably further uses the external detection tomographic image for forming the photoacoustic image. In this configuration, the photoacoustic image can be formed by using the internal detection tomographic image of the photoacoustic wave detected inside the test object and the external detection tomographic image of the photoacoustic wave detected at a surface of the test object. In the internal detection tomographic image, an intensity in detecting the photoacoustic wave generated at a deep area inside the test object is high. In the external detection tomographic image, an intensity in detecting the photoacoustic wave generated in a region near the surface of the test object is high. This makes it possible to acquire a clear photoacoustic image of a higher S/N ratio as viewed in a direction of the detection depth of the photoacoustic wave.

In this case, the internal detecting portion preferably has a detection surface provided near the internal light source and used for detecting a photoacoustic wave, and the photoacoustic wave detector preferably further includes a reflective portion having a reflective surface on which light generated from the light-emitting element is reflected toward a direction of the detection target facing the detection surface. This configuration allows light generated from the light-emitting element of the endoscope body to be emitted directly to the test object. This eliminates the need for a configuration conventionally required for guiding light from a light source provided separately from the endoscope body to the endoscope body, thereby contributing to simplification of the configuration of a light transmission path. Further, unlike in a conventional case of using a solid-state laser, using the light-emitting element such as a light-emitting diode element can contribute to size reduction of the configuration of a light source. Further, the external light source becomes usable as a light source auxiliary to the internal light source, so that light of a high intensity can be emitted to the detection target, compared to emission of light only from the internal light source. As a result, compared to emission of light only from the internal light source, a larger photoacoustic wave can be generated, so that a resultant photoacoustic image can be given a higher degree of clearness. Additionally, photoacoustic images are acquired both from the internal detecting portion and the external detecting portion. Thus, a resultant photoacoustic image is allowed to cover a wider range of the test object. Further, as a result of provision of the reflective portion to the endoscope body, emitted light not to travel in the direction of the detection target can be reflected on the reflective portion toward the detection target. This allows efficient emission of light to the detection target.

In the aforementioned configuration where the photoacoustic wave detector further includes the reflective portion, the photoacoustic imaging apparatus preferably further includes a signal processing portion. The signal processing portion is preferably configured to superimpose photoacoustic images based on corresponding photoacoustic waves detected by the internal detecting portion and the external detecting portion respectively. In this configuration, photoacoustic images acquired from corresponding photoacoustic waves detected by two different detecting portions respectively are superimposed. This makes it possible to reduce a noise component in a detection signal relatively to a signal component in the detection signal forming a photoacoustic image. As a result, a resultant photoacoustic image can be given a still higher degree of clearness.

### Advantageous Effects of Invention

As described above, the present invention is capable of detecting a photoacoustic wave of an intensity sufficient for generating a tomographic image of a deep area inside a test object by using a relatively low-output light source.

### Brief Description of Drawings

FIG. 1 is an outside perspective view showing an example of a photoacoustic imaging apparatus.
FIG. 2 is a schematic view showing an example of detection of a photoacoustic wave inside a test object.
FIG. 3 is a block diagram showing an example of the configuration of the photoacoustic imaging apparatus.
FIG. 4 is a view showing an example of an inserted part of an ultrasonic endoscope, a cross section taken along a line 610-610, and a cross section taken along a line 615-615 according to a first embodiment of the present invention.
FIG. 5 is a configuration view showing an example of the inserted part of the ultrasonic endoscope and a cross section taken along a line 620-620 according to a second embodiment of the present invention.
FIG. 6 is a configuration view showing an example of the inserted part of the ultrasonic endoscope and a cross section taken along a line 625-625 according to a third embodiment of the present invention.
FIG. 7 is a configuration view showing an example of the inserted part of the ultrasonic endoscope and a cross section taken along a line 630-630 according to a fourth embodiment of the present invention.
FIG. 8 is a configuration view showing a different example of the inserted part of the ultrasonic endoscope and a cross section taken along a line 635-635 according to the fourth embodiment of the present invention.
FIG. 9 is a configuration view showing an example of the inserted part of the ultrasonic endoscope and a cross section taken along a line 640-640 according to a fifth embodiment of the present invention.
FIG. 10 is an outside perspective view showing a different example of the photoacoustic imaging apparatus according to a sixth embodiment of the present invention.
FIG. 11A is a schematic view showing a different example of detection of a photoacoustic wave inside the test object and at a surface of the test object.
FIG. 11B is a schematic view showing a different example of detection of a photoacoustic wave inside the test object and at a surface of the test object.
FIG. 12 is a block diagram showing a different example of the configuration of the photoacoustic imaging apparatus.
FIG. 13 shows an example of arrangement of external light sources provided in a first light-emitting portion.
FIG. 14 is a block diagram showing the overall configuration of a photoacoustic endoscope according to a seventh embodiment of the present invention.
FIG. 15 is a schematic view showing a state where an endoscope body of the photoacoustic endoscope according to the seventh embodiment of the present invention is inserted into the test object.
FIG. 16 is a plan view showing the endoscope body of the photoacoustic endoscope according to the seventh embodiment of the present invention.
FIG. 17 is a sectional view taken along a line 650-650 of FIG. 16.
FIG. 18 is a sectional view taken along a line 660-660 of FIG. 16.
FIG. 19 is a plan view showing an endoscope body of a photoacoustic endoscope according to an eighth embodiment of the present invention.
FIG. 20 is a sectional view taken along a line 670-670 of FIG. 19.
FIG. 21 is a sectional view taken along a line 680-680 of FIG. 19.
FIG. 22 is a block diagram showing the overall configuration of a photoacoustic endoscope system according to a ninth embodiment of the present invention.

### Description of Embodiments

Embodiments of the present invention will be described below by referring to the drawings.

### [First Embodiment]

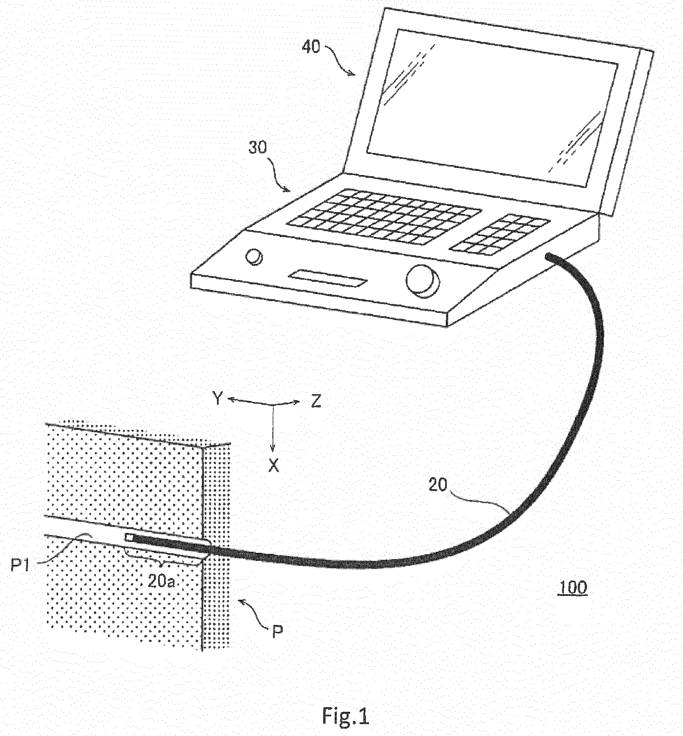

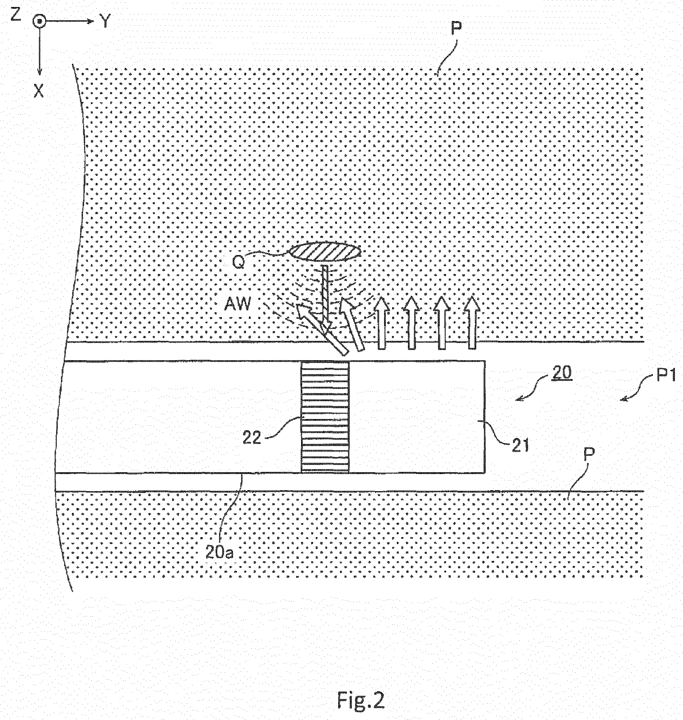

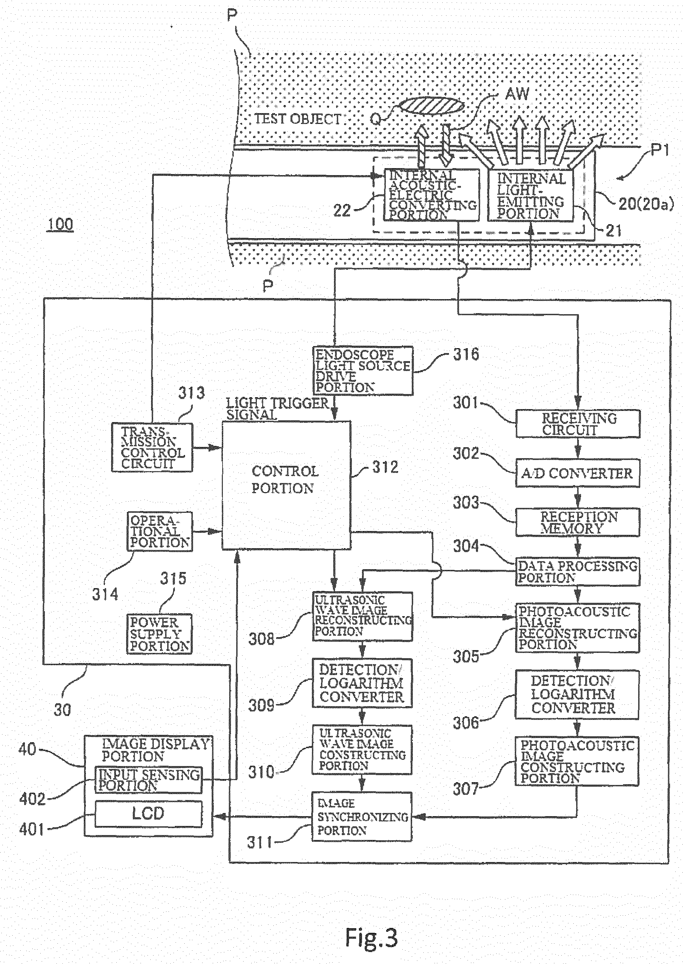

FIG. 1 is an outside perspective view showing an example of a photoacoustic imaging apparatus 100. FIG. 2 is a schematic view showing an example of detection of a photoacoustic wave AW inside a test object P. FIG. 3 is a block diagram showing an example of the configuration of the photoacoustic imaging apparatus 100.

As shown in FIG. 1, the photoacoustic imaging apparatus 100 includes a radial-type ultrasonic endoscope 20, an image generating portion 30, and an image display portion 40.

The ultrasonic endoscope 20 is a photoacoustic wave detector capable of being inserted into a body cavity P1 of the test object P such as a biological body. As shown in FIG. 2, the ultrasonic endoscope 20 has the function to be fulfilled in the body cavity P1 of emitting light to the test object P and detecting the photoacoustic wave AW generated at a detection target Q in the test object P. If the ultrasonic endoscope 20 is inserted in a direction Y into the body cavity P1, the ultrasonic endoscope 20 fulfills this function to acquire tomographic image information about the test object P taken along a plane (a plane including a direction X and a direction Z) crossing a lateral peripheral surface of an inserted part 20a at the tip of the ultrasonic endoscope 20. The ultrasonic endoscope 20 further has the function to be fulfilled in the body cavity P1 of transmitting an ultrasonic wave to the test object P and detecting an ultrasonic wave resulting from reflection of the transmitted ultrasonic wave.

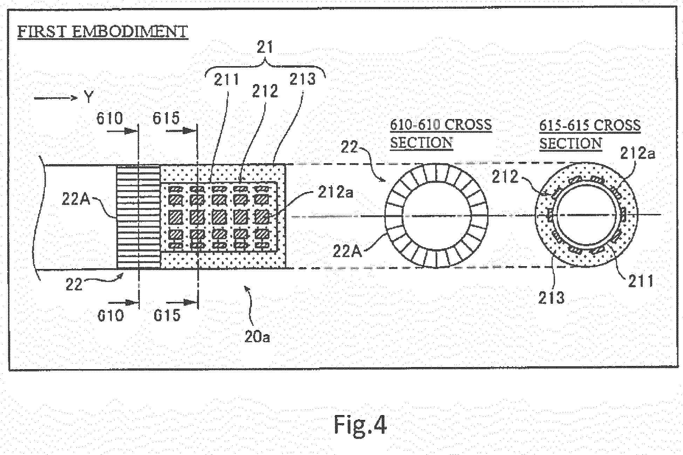

As shown in FIG. 3, the ultrasonic endoscope 20 includes an internal light-emitting portion 21 and an internal acoustic-electric converting portion 22. The internal light-emitting portion 21 has a light source including an LED (light-emitting diode) element 212a (see FIG. 4 to be referred to later for this light-emitting diode element). The internal acoustic-electric converting portion 22 is an internal detecting portion that detects elastic waves such as the photoacoustic wave AW generated at the detection target Q and an ultrasonic wave reflected in the test object P. The internal acoustic-electric converting portion 22 generates a detection signal based on a result of the detection and outputs the detection signal to the image generating portion 30. In the below, this detection signal will be called an internal detection signal.

The ultrasonic endoscope 20 emits light from the LED element 212a in the body cavity P1 inside the test object P and detects the photoacoustic wave AW also in the body cavity P1. Thus, even if the detection target Q is at a deep area inside the test object P, the ultrasonic endoscope 20 is still allowed to emit light and detect the photoacoustic wave AW at a position relatively close to the detection target Q and to form a photoacoustic image of the detection target Q. This makes it possible to detect the photoacoustic wave AW of an intensity sufficient for generating a tomographic image of the deep area inside the test object P and to form a photoacoustic image of the detected photoacoustic wave AW by using a relatively low-output light source.

The configuration of the ultrasonic endoscope 20 will be described further in detail later.

The image generating portion 30 executes imaging by processing an internal detection signal output from the ultrasonic endoscope 20. For example, the image generating portion 30 generates a photoacoustic image using tomographic image information corresponding to an internal detection signal generated based on a result of detection of the photoacoustic wave AW. The image generating portion 30 generates an ultrasonic wave image using tomographic image information corresponding to an internal detection signal generated based on a result of detection of an ultrasonic wave.

As shown in FIG. 3, the image generating portion 30 includes a receiving circuit 301, an A/D converter 302, a reception memory 303, a data processing portion 304, a photoacoustic image reconstructing portion 305, a detection/logarithm converter 306, a photoacoustic image constructing portion 307, an ultrasonic wave image reconstructing portion 308, a detection/logarithm converter 309, an ultrasonic wave image constructing portion 310, an image synthesizing portion 311, a control portion 312, a transmission control circuit 313, an operational portion 314, a power supply portion 315, and an endoscope light source drive portion 316.

The receiving circuit 301 executes processing of selecting at least some of a plurality of ultrasonic vibrating elements 22A (described later) belonging to the internal acoustic-electric converting portion 22 and amplifying a voltage signal (internal detection signal) about the selected ultrasonic vibrating element 22A.

The A/D converter 302 converts an amplified detection signal output from the receiving circuit 301 to a digital signal. The reception memory 303 stores the digital signal from the A/D converter 302. The data processing portion 304 has the function of directing the signal stored in the reception memory 303 to the photoacoustic image reconstructing portion 305 or the ultrasonic wave image reconstructing portion 308.

The photoacoustic image reconstructing portion 305 executes phase matching addition processing based on a detection signal about the photoacoustic wave AW to reconstruct data about the photoacoustic wave AW. The detection/logarithm converter 306 executes logarithmic compression processing and envelop detection processing on the reconstructed data about the photoacoustic wave AW. The photoacoustic image constructing portion 307 converts data resulting from the processing by the detection/logarithm converter 306 to brightness value data in units of pixels. Specifically, photoacoustic image data (gray scale, for example) is generated as brightness value data in units of pixels of an image in a vertical direction and a horizontal direction.

This photoacoustic image data is data indicating a photoacoustic image formed by executing averaging processing on a tomographic image based on a detection signal about the photoacoustic wave AW. Each pixel in the photoacoustic image shows a detection level of the photoacoustic wave AW after the averaging processing in terms of a 256-shades gray scale, for example.

The photoacoustic image reconstructing portion 305, the detection/logarithm converter 306, the photoacoustic image constructing portion 307, and the image synthesizing portion 311 are examples of an internal detection image generating portion and an image forming portion according to the present invention and responsible for imaging processing on the photoacoustic wave AW.

The ultrasonic wave image reconstructing portion 308 executes phase matching addition processing based on a detection signal about an ultrasonic wave to reconstruct data about the ultrasonic wave. The detection/logarithm converter 309 executes logarithmic compression processing and envelop detection processing on the reconstructed data about the ultrasonic wave. The ultrasonic wave image constructing portion 310 converts data resulting from the processing by the detection/logarithm converter 309 to brightness value data in units of pixels. Specifically, ultrasonic wave image data (gray scale, for example) is generated as brightness value data in units of pixels of an image in a vertical direction and a horizontal direction.

The image synthesizing portion 311 synthesizes the aforementioned photoacoustic image data and the aforementioned ultrasonic wave image data to generate synthetic image data. The image synthesis mentioned herein may be made by superimposing the photoacoustic image on the ultrasonic wave image or by arranging the photoacoustic image and the ultrasonic wave image side by side. The image display portion 40 displays an image based on the synthetic image data generated by the image synthesizing portion 311. The image synthesizing portion 311 may output the photoacoustic image data or the ultrasonic wave image data as it is to the image display portion 40.

The control portion 312 controls each structural element of the photoacoustic imaging apparatus 100 using a program and control information, etc. stored in a non-transitory storage medium (memory) not shown in the drawings. For example, the control portion 312 transmits a light trigger signal to the endoscope light source drive portion 316.

The transmission control circuit 313 transmits a drive signal to the internal acoustic-electric converting portion 22 to make the internal acoustic-electric converting portion 22 generate an ultrasonic wave in response to a control signal output from the control portion 312.

The operational portion 314 accepts operational input from a user and outputs an input signal responsive to the operational input to the control portion 312.

The power supply portion 315 supplies power to each structural element of the photoacoustic imaging apparatus 100.

The endoscope light source drive portion 316 is a drive circuit portion that drives a light source of the internal light-emitting portion 21 of the ultrasonic endoscope 20. For example, in response to receipt of a light trigger signal from the control portion 312, the endoscope light source drive portion 316 transmits a drive signal to the internal light-emitting portion 21. If the internal light-emitting portion 21 includes several types of light sources for emitting pulsed light of different wavelengths as described later, a wavelength control signal is output from the control portion 312. In this case, in response to receipt of the wavelength control signal from the control portion 312, the endoscope light source drive portion 316 selects a light source in response to the wavelength control signal and drives the selected light source.

The image display portion 40 will be described next. The image display portion 40 is a display device having a touch panel. As shown in FIG. 3, the image display portion 40 includes an LCD (liquid crystal display) 401 and an input sensing portion 402. The LCD 401 displays an image (a photoacoustic image, for example) generated by the image generating portion 30 based on an image signal. The input sensing portion 402 accepts operational input from a user. If a touch input is given on a display screen on the LCD 401 with a finger of the user or a stylus pen, for example, the input sensing portion 402 outputs an input signal responsive to the touch input to the control portion 312.

The configuration of the ultrasonic endoscope 20 will be described in detail next. FIG. 4 is a configuration view showing an example of the inserted part 20a of the ultrasonic endoscope 20 according to the first embodiment. A 610-610 cross-sectional view of FIG. 4 shows a cross section of the inserted part 20a taken along a line 610-610. A 615-615 cross-sectional view of FIG. 4 shows a cross section of the inserted part 20a taken along a line 615-615.

As shown in FIG. 4, the inserted part 20a to be inserted into the body cavity P1 of the test object P includes the internal light-emitting portion 21 and the internal acoustic-electric converting portion 22.

The internal light-emitting portion 21 includes a substrate 211, an internal light source 212, and a sealing layer 213. The substrate 211 is a flexible substrate, for example, and is formed into a cylindrical shape having an axis parallel to a direction of insertion of the inserted part 20a (direction Y). The substrate 211 is provided at the tip of the inserted part 20a. For example, the substrate 211 is provided concentrically with the lateral peripheral surface of the inserted part 20a in a plan view taken in the direction of insertion. The substrate 211 is not limited to the illustration of FIG. 4 but it may also be a substrate having a circular cylindrical shape or a circular columnar shape (a print substrate or a metal substrate, for example).

The internal light source 212 includes a plurality of LED elements 212a to be inserted into the test object P and applies light emitted from the LED elements 212a to the inside of the test object P (particularly, to the detection target Q). The LED elements 212a are arranged on a main surface of the substrate 211 in the direction of insertion and along the lateral peripheral surface of the substrate 211 to be capable of emitting light uniformly in every direction of a normal to the lateral peripheral surface of the inserted part 20a. Thus, a surface on which the LED elements 212a are mounted is a curved surface concentric with the lateral peripheral surface of the inserted part 20a as viewed in the direction Y. In FIG. 4, the LED elements 212a on the substrate 211 are arranged in five lines in the axis direction and 10 lines in the circumferential direction (specifically, 50 LED elements 212a are arranged), for example. Specifically, the LED elements 212a are arranged to be pointed outward of the direction of the normal to the surface of the inserted part 20a on which the LED elements 212a are mounted. In this way, as viewed in the direction of insertion of the inserted part 20a, the LED elements 212a are configured to emit light outward of the direction of the normal to the mounting surface so as to cover the entire periphery of the mounting surface. The internal light source 212 is provided near the tip of the inserted part 20a to be inserted into the test object P.

The LED elements 212a are LED light sources driven to emit pulsed light. The PRF (pulse repetition frequency) of the pulsed light is not particularly limited but it can be set at 1000 [times/sec], for example. Using the LED light sources allows size reduction of the internal light source 212, so that the pulsed light of a high light emission frequency can be emitted to the test object P using a simple configuration. This makes it possible to acquire more tomographic image information per unit time (a period described later required for updating display on the LCD 401, for example). As a result, a photoacoustic image can be given a higher degree of clearness and higher resolution.

The LED elements 212a may be formed of LED light sources having the same light emission wavelength or several types of LED light sources having different light emission wavelengths. The light emission wavelength of the LED elements 212a can be set by selecting a wavelength producing a high index of absorption by a measurement target (detection target Q). If the measurement target is oxyhemoglobin in blood, for example, 760 nm producing a high index of absorption by oxyhemoglobin can be selected as a light emission wavelength. If the measurement target is reduced hemoglobin in blood, for example, 850 nm producing a high index of absorption by reduced hemoglobin can be selected as a light emission wavelength. If pulsed light of a wavelength of 760 nm is emitted to the test object P, for example, oxyhemoglobin in blood contained in an arterial vessel, a tumor, etc. in the test object P absorbs the light to generate the photoacoustic wave AW (see FIG. 3). Then, the photoacoustic image constructing portion 307 generates a photoacoustic image described later including the arterial vessel, the tumor, etc.

The sealing layer 213 seals the substrate 211 and the LED elements 212a. A material for the sealing layer 213 is not particularly limited but it is required only to have light-transmitting properties of letting at least light emitted from the LED elements 212a pass through. For example, the sealing layer 213 may be formed by using glass, a light-transmitting resin material, or a light-emitting composite resin material including a filler.

The internal acoustic-electric converting portion 22 is provided on the lateral peripheral surface of the inserted part 20a to be inserted into the test object P in such a manner that the internal acoustic-electric converting portion 22 is located at a position behind and near the internal light-emitting portion 21 as viewed in the direction of insertion. As viewed in the direction of insertion of the inserted part 20a, the internal acoustic-electric converting portion 22 is provided along the outer periphery of the lateral surface of the inserted part 20a. The internal acoustic-electric converting portion 22 is provided near the tip of the inserted part 20a to be inserted into the test object P. The internal acoustic-electric converting portion 22 includes the ultrasonic vibrating element 22A, an acoustic lens (not shown in the drawings), an acoustic matching layer (not shown in the drawings), and a backing member (not shown in the drawings).

The ultrasonic vibrating element 22A is a piezoelectric element that vibrates to generate an ultrasonic wave in response to application of a voltage and generates a voltage in response to application of vibration (ultrasonic wave). As shown in FIG. 4, the ultrasonic vibrating element 22A includes a plurality of ultrasonic vibrating elements 22A arranged over the entire periphery of the lateral peripheral surface of the inserted part 20a. This allows the ultrasonic endoscope 20 to acquire photoacoustic image information covering a wide range along the entire periphery of the lateral peripheral surface of the inserted part 20a.

The acoustic matching layer is provided on all the outer surfaces of the ultrasonic vibrating elements 22A to extend along the entire periphery of the lateral peripheral surface. The acoustic lens is provided entirely on the acoustic matching layer. The backing member for suppressing propagation of an ultrasonic wave is provided on all the inner surfaces of the ultrasonic vibrating elements 22A. The acoustic matching layer reduces a difference in an acoustic impedance between the test object P and the ultrasonic vibrating elements 22A. For example, the acoustic matching layer has the function of propagating an ultrasonic wave generated from the ultrasonic vibrating element 22A efficiently into the test object P. The acoustic matching layer further has the function of propagating an ultrasonic wave (including the photoacoustic wave AW) from the test object P efficiently to the ultrasonic vibrating element 22A. The acoustic lens has the function of focusing an ultrasonic wave output from the ultrasonic vibrating element 22A.

### [Second Embodiment]

A second embodiment will be described next. The following description is about structures different from those of the first embodiment. Structures similar to those of the first embodiment will be given the same signs and in some cases, description of such structures will be omitted.

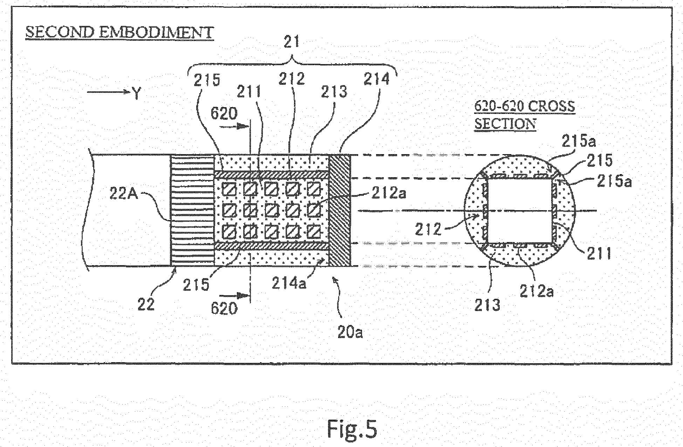

FIG. 5 is a configuration view showing an example of the inserted part 20a of the ultrasonic endoscope 20 according to the second embodiment. A 620-620 cross sectional view of FIG. 5 shows a cross section of the inserted part 20a taken along a line 620-620.

As shown in FIG. 5, the substrate 211 has an axis parallel to the direction of insertion of the inserted part 20a and is formed into a tubular shape square in cross section. The shape of the substrate 211 is not limited to the illustration of FIG. 5 but it may also be a columnar shape polygonal in cross section (triangular or hexagonal, for example) other than square.

The LED element 212a is arranged on each side surface of the substrate 211 facing the lateral peripheral surface of the inserted part 20a to be capable of emitting light in a direction of a normal to this side surface (a surface on which the LED element 212a is mounted). In the illustration of FIG. 5, the LED elements 212a in five rows and three lines (specifically, 15 LED elements 212a) are arranged on each side surface of the substrate 211. The LED elements 212a of the same light emission wavelength may be arranged on every side surface of the substrate 211. Alternatively, the light emission wavelength of the LED elements 212a may differ among all the side surfaces. Each side surface of the substrate 211 extends in the direction of insertion of the inserted part 20a.

The internal light-emitting portion 21 includes a reflective portion 214 and four reflective plates 215 in addition to the substrate 211, the internal light source 212, and the sealing layer 213.

The reflective portion 214 is a reflective member having a surface pointed to the LED element 212a and functioning as a reflective surface 214a on which light emitted from the LED element 212a is reflected. The reflective portion 214 is located at a position of the inserted part 20a ahead of the substrate 211 (in front of the substrate 211 as viewed in the direction of insertion).

Each of the reflective plates 215 is a reflective member having opposite main surfaces each functioning as a reflective surface 215a on which light emitted from the LED element 212a is reflected. Each reflective plate 215 extends in a direction from an edge of a corresponding side surface of the substrate 211 parallel to the direction of insertion toward the lateral peripheral surface of the inserted part 20a.

The reflective surface 214a of the reflective portion 214 and the reflective surface 215a of each reflective plate 215 are both allowed to cause reflection of light emitted from the LED element 212a and traveling in a direction toward the direction of insertion and to cause reflection of the reflected light in a direction toward a direction of the normal to the outer peripheral surface of the internal acoustic-electric converting portion 22. Specifically, the reflective portion 214 is configured to reflect light emitted from the LED element 212a outward of the direction of the normal to the mounting surface. This achieves efficient emission of light to the test object P. This further makes it possible to emit light easily to a region in the vicinity of the outer peripheral surface of the internal acoustic-electric converting portion 22. Thus, even if the detection target Q is near the internal acoustic-electric converting portion 22, the photoacoustic wave AW of a sufficient intensity can still be detected from the detection target Q.

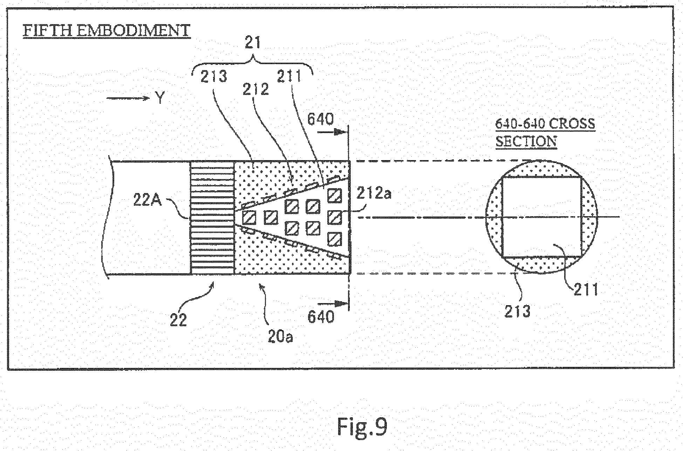

The aforementioned configuration including the reflective surfaces 214a and 215a is applicable not only to the configuration of this embodiment but also to the configurations of other embodiments (a configuration of FIG. 9 referred to later, for example).

### [Third Embodiment]

A third embodiment will be described next. The following description is about structures different from those of the first and second embodiments. Structures similar to those of the first and second embodiments will be given the same signs and in some cases, description of such structures will be omitted.

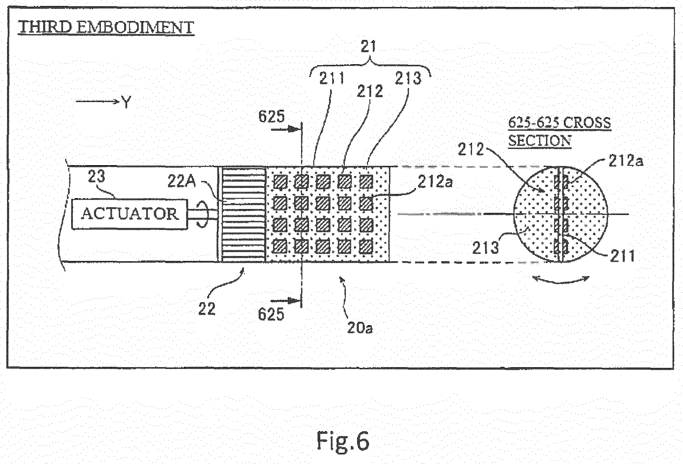

FIG. 6 is a configuration view showing an example of the inserted part 20a of the ultrasonic endoscope 20 according to the third embodiment. A 625-625 cross sectional view of FIG. 6 shows a cross section of the inserted part 20a taken along a line 625-625.

As shown in FIG. 6, the internal light-emitting portion 21 includes the substrate 211, the internal light source 212, and the sealing layer 213. In the internal light-emitting portion 21, the substrate 211 is arranged parallel to the direction of insertion of the inserted part 20a. Further, the LED elements 212a are arranged on the opposite main surfaces of the substrate 211. In the illustration of FIG. 6, the LED elements 212a in five rows and four lines (specifically, 20 LED elements 212a) are arranged on each main surface of the substrate 211. Further, as shown in FIG. 5 corresponding to the second embodiment, the internal light-emitting portion 21 may include a reflective member (reflective portion 214, for example). The reflective member has a surface facing the LED element 212a and functioning as the reflective surface 214a on which light emitted from the LED element 212a is reflected, and is located at a position of the inserted part 20a in front of the substrate 211 as viewed in the direction of insertion.

As shown in FIG. 6, the ultrasonic endoscope 20 includes an actuator 23. The actuator 23 is a rotation drive portion capable of rotating the inserted part 20a about the direction of insertion as an axis based on a control signal output from the control portion 312.

In FIG. 6, the inserted part 20a is rotatable about the direction of insertion of the inserted part 20a as an axis. This makes it possible to change a direction of application of pulsed light emitted from the LED element 212a to any direction perpendicular to the direction of insertion of the inserted part 20a. As a result, a measurement region to appear in a tomographic image can be changed arbitrarily.

The LED elements 212a of the same light emission wavelength may be arranged on both of the opposite main surfaces of the substrate 211. However, the light emission wavelength of the LED elements 212a preferably differs between the main surfaces. In this case, the wavelength of pulsed light to be applied to an intended measurement region can be changed by the rotation of the inserted part 20a. As a result, the wavelength of pulsed light to be emitted for forming a photoacoustic image can be changed according to the type of the detection target Q.

### [Fourth Embodiment]

A fourth embodiment will be described next. The following description is about structures different from those of the first to third embodiments. Structures similar to those of the first to third embodiments will be given the same signs and in some cases, description of such structures will be omitted.

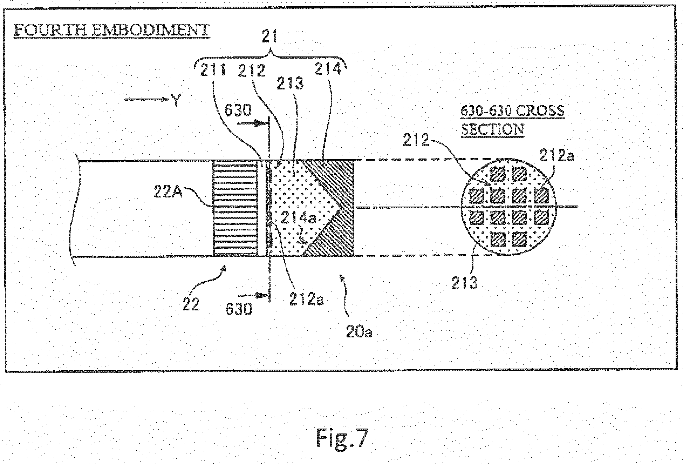

FIG. 7 is a configuration view showing an example of the inserted part 20a of the ultrasonic endoscope 20 according to the fourth embodiment. A 630-630 cross sectional view of FIG. 7 shows a cross section of the inserted part 20a taken along a line 630-630.

In the internal light-emitting portion 21, the substrate 211 is provided ahead of the internal acoustic-electric converting portion 22 to cross the direction of insertion of the inserted part 20a. The LED element 212a is arranged on a main surface of the substrate 211 pointed to the direction of insertion and emits pulsed light in a direction toward the direction of insertion.

As shown in FIG. 7, the internal light-emitting portion 21 includes the reflective portion 214 in addition to the substrate 211, the internal light source 212, and the sealing layer 213. The reflective portion 214 is a reflective member having a surface pointed to the LED element 212a and functioning as the reflective surface 214a on which light emitted from the LED element 212a is reflected. The reflective portion 214 is located at a position of the inserted part 20a in front of the substrate 211 as viewed in the direction of insertion. The reflective surface 214a is allowed to cause reflection of light emitted from the LED element 212a and traveling in a direction toward the direction of insertion and to cause reflection of the reflected light in a direction toward a direction of a normal to the outer peripheral surface of the internal acoustic-electric converting portion 22. Specifically, the reflective portion 214 is configured to reflect light emitted from the LED element 212a outward of a direction of a normal to the mounting surface. This achieves efficient emission of light to the test object P. This further makes it possible to emit light easily to a region in the vicinity of the outer peripheral surface of the internal acoustic-electric converting portion 22. Thus, even if the detection target Q is near the internal acoustic-electric converting portion 22, the photoacoustic wave AW of a sufficient intensity can still be detected from the detection target Q.

### [Different Example of Fourth Embodiment]

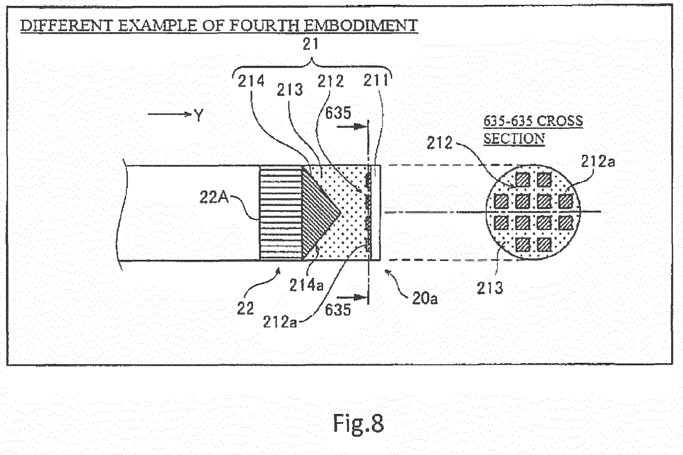

The arrangement of the LED element 212a and that of the reflective portion 214 may be reversed as viewed in the direction of insertion. FIG. 8 is a configuration view showing a different example of the inserted part of the ultrasonic endoscope according to the fourth embodiment. A 635-635 cross sectional view of FIG. 8 shows a cross section of the inserted part 20a taken along a line 635-635.

In FIG. 8, the substrate 211 is provided at the tip of the inserted part 20a of the ultrasonic endoscope 20 and the reflective portion 214 is provided behind the substrate 211 as viewed in the direction of insertion. The LED element 212a is arranged on a main surface of the substrate 211 facing the reflective surface 214a of the reflective portion 214. The reflective surface 214a is allowed to cause reflection of light emitted from the LED element 212a and traveling in a direction toward the direction of insertion and to cause reflection of the reflected light in a direction toward a direction of a normal to the outer peripheral surface of the internal acoustic-electric converting portion 22. This configuration still achieves effect comparable to that achieved by the configuration of FIG. 7.

### [Fifth Embodiment]

A fifth embodiment will be described next. The following description is about structures different from those of the first to fourth embodiments. Structures similar to those of the first to fourth embodiments will be given the same signs and in some cases, description of such structures will be omitted.

FIG. 9 is a configuration view showing an example of the inserted part 20a of the ultrasonic endoscope 20 according to the fifth embodiment. A 640-640 cross sectional view of FIG. 9 shows a cross section of the inserted part 20a taken along a line 640-640.

As shown in FIG. 9, the substrate 211 has a frustum shape having an axis parallel to the direction of insertion of the inserted part 20a and having a square cross section. Each side surface of this frustum shape is tilted toward the internal acoustic-electric converting portion 22. The shape of the substrate 211 is not limited to the illustration of FIG. 9 but it may also be a pointed shape such as a conical shape or a pyramid shape. The cross-sectional shape of the substrate 211 perpendicular to the direction of insertion may be polygonal (triangular or hexagonal, for example) other than circular or square.

The LED element 212a is arranged on each side surface of the substrate 211 facing the lateral peripheral surface of the inserted part 20a to be capable of emitting light in a direction of a normal to this side surface (a surface on which the LED element 212a is mounted). In the illustration of FIG. 9, nine LED elements 212a are arranged on each side surface of the substrate 211. The LED elements 212a of the same light emission wavelength may be arranged on every side surface of the substrate 211. Alternatively, the light emission wavelength of the LED elements 212a may differ among all the side surfaces.

In FIG. 9, the LED element 212a is arranged on the side surface of the substrate 211 tilted toward the internal acoustic-electric converting portion 22. This makes it possible to direct light emitted from the LED element 212a toward the detection target Q existing in a direction of a normal to the outer peripheral surface of the internal acoustic-electric converting portion 22. This further makes it possible to emit light easily to a region in the vicinity of the outer peripheral surface of the internal acoustic-electric converting portion 22. Thus, even if the detection target Q is near the internal acoustic-electric converting portion 22, the photoacoustic wave AW of a sufficient intensity can still be detected from the detection target Q.

### [Sixth Embodiment]

A sixth embodiment will be described next. According to the sixth embodiment, the photoacoustic imaging apparatus 100 further includes an ultrasonic probe 50 for acquiring tomographic image information at a surface of the test object P. The photoacoustic imaging apparatus 100 achieves photoacoustic imaging and ultrasonic imaging using both the ultrasonic endoscope 20 and the ultrasonic probe 50. The sixth embodiment is the same in the other respects as the first to fifth embodiments. The following description is about structures different from those of the first to fifth embodiments. Structures similar to those of the first to fifth embodiments will be given the same signs and in some cases, description of such structures will be omitted.

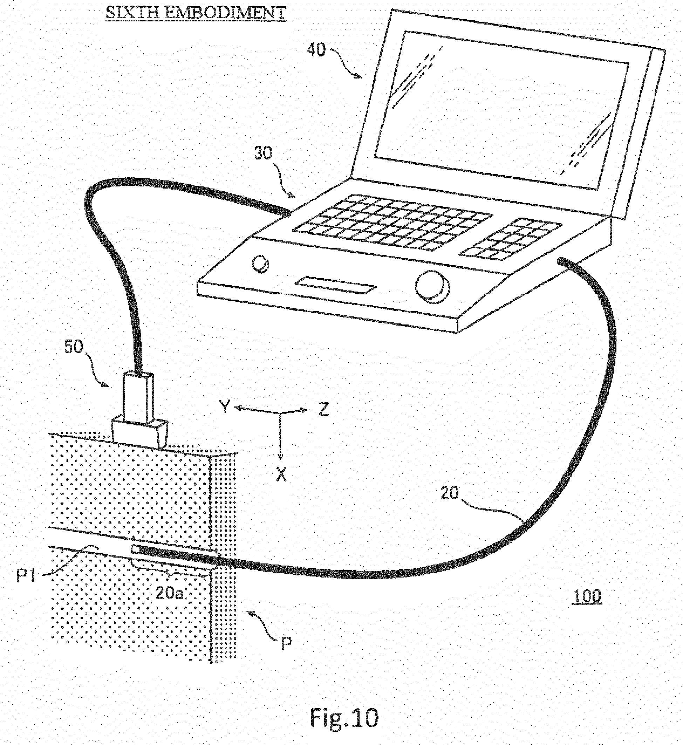

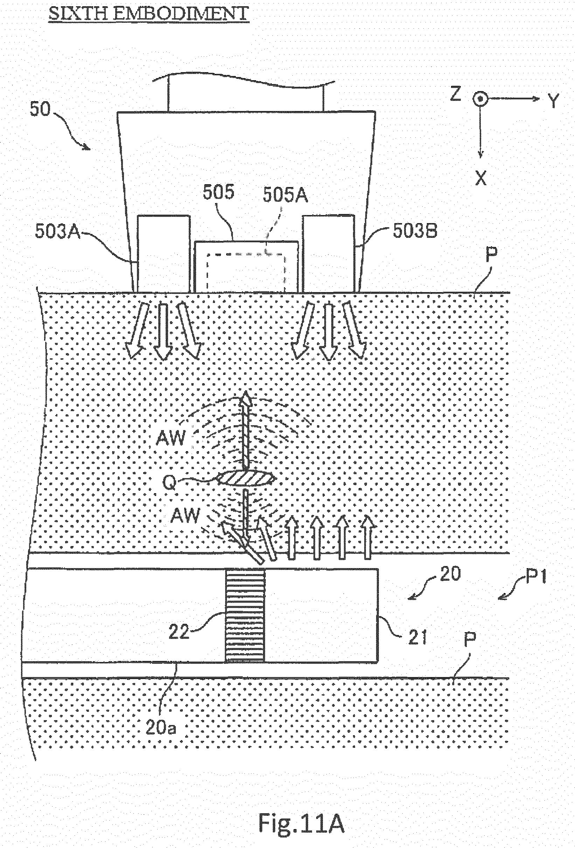

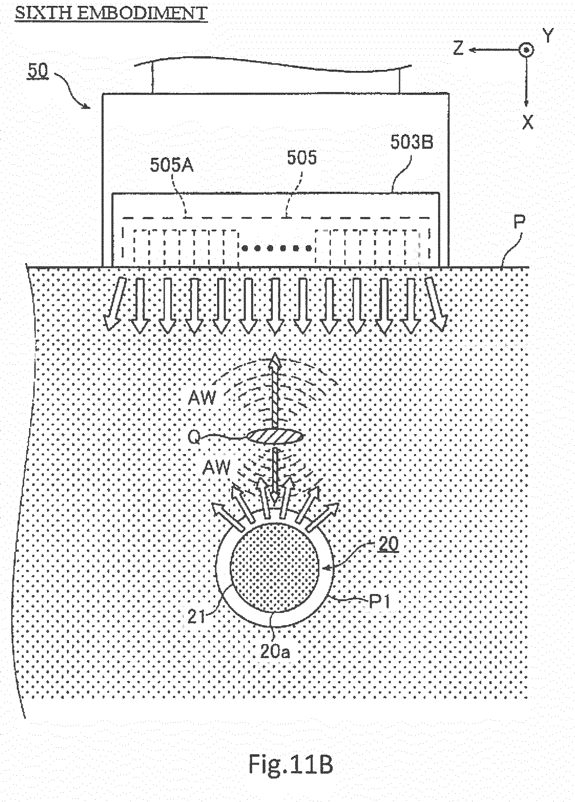

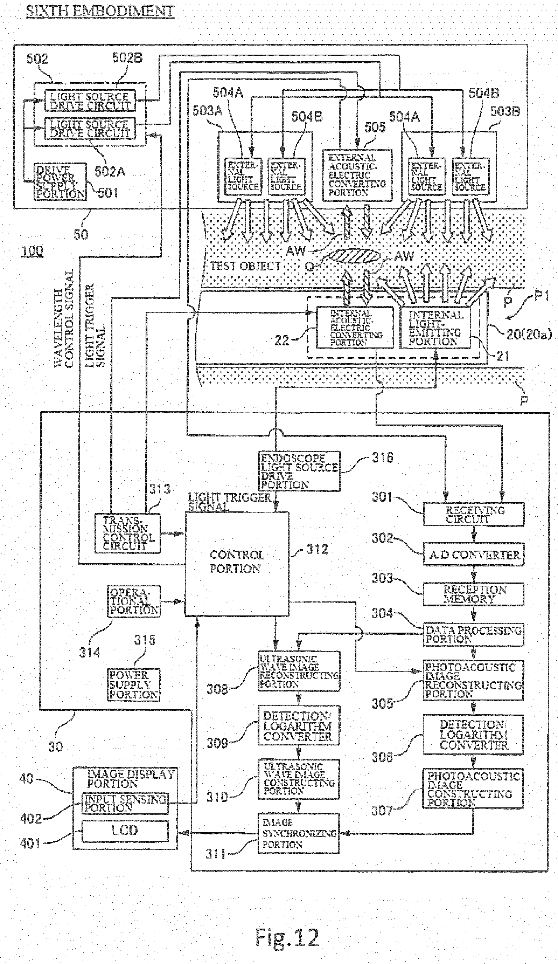

FIG. 10 is an outside perspective view showing a different example of the photoacoustic imaging apparatus 100. FIGS. 11A and 11B are schematic views each showing a different example of detection of the photoacoustic wave AW inside the test object P and at a surface of the test object P. FIG. 11A is a schematic view taken in the direction Z. FIG. 11B is a schematic view taken in the direction Y. FIG. 12 is a block diagram showing a different example of the configuration of the photoacoustic imaging apparatus 100.

As shown in FIG. 10, the photoacoustic imaging apparatus 100 includes the ultrasonic probe 50 in addition to the radial-type ultrasonic endoscope 20, the image generating portion 30, and the image display portion 40. As shown in FIGS. 11A and 11B, the ultrasonic probe 50 has the function of emitting light from a surface of the test object P and detecting the photoacoustic wave AW generated at the detection target Q in the test object P. The ultrasonic probe 50 further has the function of transmitting an ultrasonic wave to the test object P and detecting an ultrasonic wave resulting from reflection of the transmitted ultrasonic wave.

As shown in FIG. 12, the ultrasonic probe 50 includes a drive power supply portion 501, a light source drive portion 502, a first light-emitting portion 503A and a second light-emitting portion 503B each having an external light source 504A and an external light source 504B, and an external acoustic-electric converting portion 505. The ultrasonic probe 50 may further include a lens member to collect light emitted from the external light sources 504A and 504B and a light guide portion (a light guide plate or an optical fiber made of acrylic resin, for example) to guide the light collected by the lens member to the test object P.

The drive power supply portion 501 supplies power to the light source drive portion 502. In the illustration of FIG. 12, the drive power supply portion 501 is a power supply for the ultrasonic probe 50. However, this illustration is not restrictive but each structural element of the ultrasonic probe 50 may receive power supplied from the power supply portion 315.

The light source drive portion 502 includes a light source drive circuit 502A and a light source drive circuit 502B. The light source drive circuit 502A controls drive of the external light source 504A. The light source drive circuit 502B controls drive of the external light source 504B. If the ultrasonic probe 50 and the ultrasonic endoscope 20 are to perform photoacoustic imaging together to acquire tomographic images of the same measurement region, the light source drive circuits 502A and 502B respectively make the external light sources 504A and 504B emit pulsed light to conform to timing of emission of pulsed light from the ultrasonic endoscope 20. Specifically, the light source drive circuits 502A and 502B control drive of the external light sources 504A and 504B respectively so as to bring timing of emission of pulsed light from the external light sources 504A and 504B into synchronization with timing of emission of pulsed light from the ultrasonic endoscope 20.

The first and second light-emitting portions 504A and 504B are arranged to sandwich the external acoustic-electric converting portion 505 therebetween (see FIG. 11A). The first and second light-emitting portions 504A and 504B are arranged to be located near the test object P when the ultrasonic probe 50 comes into contact with the test object P.

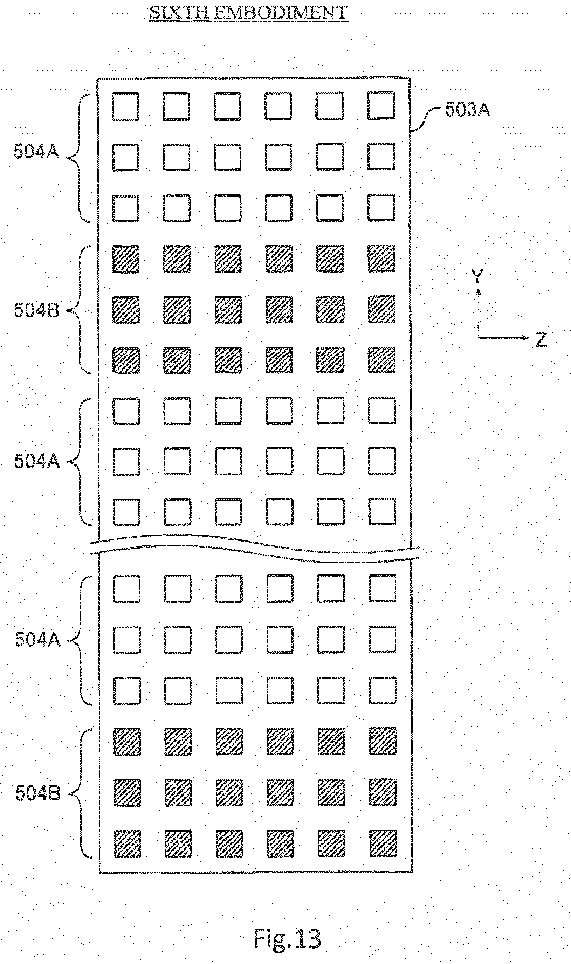

Each of the external light sources 504A and 504B provided in each of the first and second light-emitting portions 503A and 503B includes a light-emitting element such as an LED element (not shown in the drawings), for example, and emits pulsed light from a surface of the test object P to the inside of the test object P. FIG. 13 shows an example of arrangement of the external light sources 504A and 504B provided in the first light-emitting portion 503A. The following description given by referring to FIG. 13 is about an example of the arrangement in the first light-emitting portion 503A. Arrangement of the external light sources 504A and 504B provided in the second light-emitting portion 503B is the same as that in the first light-emitting portion 503A.

As shown in FIG. 13, the external light sources 504A and 504B are arranged alternately in the direction Y. Each of the external light sources 504A and 504B includes LED elements in three lines in the direction Y and six lines in the direction Z (specifically, 3 x 6 = 18 LED elements). These LED elements are LED light sources driven to emit pulsed light. The PRF (pulse repetition frequency) of the pulsed light is not particularly limited but it can be set at 1000 [times/sec], for example.

Using the LED light sources in each of the external light sources 504A and 504B allows size reduction of the external light sources 504A and 504B, so that pulsed light of a high light emission frequency can be emitted to the test object P using a simple configuration. This makes it possible to acquire more tomographic image information per unit time (a period described later required for updating display on the LCD 401, for example). As a result, a photoacoustic image described later can be given a higher degree of clearness and higher resolution.

The light emission wavelength of the LED element differs between the external light sources 504A and 504B. Specifically, the wavelength of light to be emitted differs between the external light sources 504A and 504B. Regarding a setting of a wavelength, a wavelength producing a high index of absorption by a measurement target (detection target Q) can be selected.

Referring next to the external acoustic-electric converting portion 505, the external acoustic-electric converting portion 505 is an external detecting portion that detects elastic waves such as the photoacoustic wave AW generated at the detection target Q (see FIG. 12) and an ultrasonic wave reflected in the test object P. The external acoustic-electric converting portion 505 generates a detection signal based on a result of the detection and outputs the detection signal to the image generating portion 30. In the below, this detection signal will be called an external detection signal.

The external acoustic-electric converting portion 505 includes an ultrasonic vibrating element 505A, an acoustic lens (not shown in the drawings), an acoustic matching layer (not shown in the drawings), and a backing member (not shown in the drawings).

The ultrasonic vibrating element 505A is a piezoelectric element that vibrates to generate an ultrasonic wave in response to application of a voltage and generates a voltage in response to application of vibration (ultrasonic wave). The ultrasonic vibrating element 505A generates an ultrasonic wave and propagates the ultrasonic wave into the test object P. Then, the ultrasonic vibrating element 505A detects the ultrasonic wave reflected in the test object P to generate a voltage signal. Specifically, using the ultrasonic probe 50 achieves ultrasonic imaging in addition to photoacoustic imaging.

The ultrasonic vibrating element 505A is arranged between the first and second light-emitting portions 503A and 503B (see FIG. 11A). The ultrasonic vibrating element 505A includes a plurality of ultrasonic vibrating elements 505A arranged in the direction Z (see FIG. 11B). A surface of the ultrasonic vibrating element 505A adjacent to the test object P (the lower surface of the ultrasonic vibrating element 505A shown in FIGS. 11A and 11B, for example) is provided with the acoustic matching layer and the acoustic lens arranged in order. A surface opposite this surface (the upper surface of the ultrasonic vibrating element 505A shown in FIGS. 11A and 11B, for example) is provided with the backing member for suppressing propagation of an ultrasonic wave.

Processing on the ultrasonic probe 50 executed by the image generating portion 30 will be described next. The receiving circuit 301 further executes processing of selecting at least some of the ultrasonic vibrating elements 505A and amplifying a voltage signal (external detection signal) about the selected ultrasonic vibrating element 505A.

The A/D converter 302 converts an amplified detection signal (internal detection signal or external detection signal) output from the receiving circuit 301 to a digital signal.

The photoacoustic image reconstructing portion 305, the detection/logarithm converter 306, and the photoacoustic image constructing portion 307 execute imaging processing on the photoacoustic wave AW to generate photoacoustic image data. This photoacoustic image data is data indicating a photoacoustic image formed by averaging processing on a tomographic image based on a detection signal about the photoacoustic wave AW. The photoacoustic image reconstructing portion 305, the detection/logarithm converter 306, the photoacoustic image constructing portion 307, and the image synthesizing portion 311 are examples of the internal detection image generating portion, an external detection image generating portion, and the image forming portion according to the present invention and responsible for imaging processing on the photoacoustic wave AW.

The following describes a case where both the ultrasonic endoscope 20 and the ultrasonic probe 50 are used for photoacoustic imaging. In this case, a photoacoustic image can be formed by executing averaging processing on an internal detection tomographic image based on an internal detection signal output from the ultrasonic endoscope 20 and an external detection tomographic image based on an external detection signal output from the ultrasonic probe 50. A direction of the detection depth of the photoacoustic wave AW in the external detection tomographic image is opposite a corresponding direction in the internal detection tomographic image. If a direction of the detection depth is the direction -X in the internal detection tomographic image, for example, a corresponding direction in the external detection tomographic image is the direction X. This certainly means that the averaging processing for forming a photoacoustic image is executed after processing of bringing the directions of measurement depth in both of these images into agreement.

By forming a photoacoustic image in this way, the internal detection tomographic image and the external detection tomographic image can be superimposed in the resultant photoacoustic image, for example. Specifically, the photoacoustic image can be formed by using the internal detection tomographic image of the photoacoustic wave AW detected inside the test object P and the external detection tomographic image of the photoacoustic wave AW detected at a surface of the test object P. In the internal detection tomographic image, an intensity in detecting the photoacoustic wave AW generated at a deep area inside the test object P is high. In the external detection tomographic image, an intensity in detecting the photoacoustic wave AW generated in a region near the surface of the test object P is high. This makes it possible to acquire a clear photoacoustic image of a higher S/N ratio as viewed in a direction of the detection depth of the photoacoustic wave AW.

In the aforementioned photoacoustic imaging, averaging processing is executed on an internal detection tomographic image and an external detection tomographic image to form a photoacoustic image. Meanwhile, in the photoacoustic imaging apparatus 100 according to this embodiment, an internal detection tomographic image and an external detection tomographic image can be subjected to averaging processing separately. In this case, a photoacoustic image based on an internal detection tomographic image and a photoacoustic image based on an external detection tomographic image can be formed separately.

In the photoacoustic imaging apparatus 100 according to this embodiment, a photoacoustic image can also be formed based on one of an internal detection tomographic image and an external detection tomographic image. In this case, both the ultrasonic endoscope 20 and the ultrasonic probe 50 may emit pulsed light of the same emission timing, and one of the ultrasonic endoscope 20 and the ultrasonic probe 50 may detect the photoacoustic wave AW. By doing so, if a photoacoustic image is to be formed based on an internal detection tomographic image, the external light sources 504A and 504B of the ultrasonic probe 50 existing at a surface of the test object P can be used as an auxiliary light source. If a photoacoustic image is to be formed based on an external detection tomographic image, the internal light source 212 of the ultrasonic endoscope 20 existing in the body cavity P1 can be used as an auxiliary light source. This can increase an intensity in detecting the photoacoustic wave AW to a greater degree. As a result, a resultant photoacoustic image can be given a higher degree of clearness.

Whether a photoacoustic image is to be formed based on an internal detection tomographic image and an external detection tomographic image, or photoacoustic images based on these detection tomographic images are to be formed separately, can be determined in response to operational input from a user, for example.

The ultrasonic wave image reconstructing portion 308, the detection/logarithm converter 309, and the ultrasonic wave image constructing portion 310 execute imaging processing on an ultrasonic wave reflected in the test object P to generate ultrasonic wave image data. This ultrasonic wave image data is data indicating an ultrasonic wave image formed by averaging processing on a tomographic image based on a detection signal about the ultrasonic wave. If both the ultrasonic endoscope 20 and the ultrasonic probe 50 are used for ultrasonic imaging, a photoacoustic image can be formed by executing averaging processing on an internal detection tomographic image based on an internal detection signal output from the ultrasonic endoscope 20 and an external detection tomographic image based on an external detection signal output from the ultrasonic probe 50.

The control portion 312 can control each structural element of the ultrasonic probe 50 using a program and control information, etc. stored in a non-transitory storage medium (memory) not shown in the drawings. For example, the control portion 312 transmits a wavelength control signal, a light trigger signal, etc. to the light source drive portion 502. The transmission control circuit 313 transmits a drive signal to the external acoustic-electric converting portion 505 to make the external acoustic-electric converting portion 505 generate an ultrasonic wave in response to a control signal output from the control portion 312.

As described above, the photoacoustic imaging apparatus 100 according to this embodiment is capable of forming a photoacoustic image containing superimposed tomographic image information pieces about a common measurement region by performing photoacoustic imaging by using both the ultrasonic endoscope 20 and the ultrasonic probe 50 (see FIGS. 11A and 11B). The photoacoustic imaging apparatus 100 is also capable of forming an ultrasonic wave image by performing ultrasonic imaging in the same manner. The following describes an example of photoacoustic imaging processing by the photoacoustic imaging apparatus 100 according to this embodiment.

First, a measurement position of the ultrasonic endoscope 20 in the direction Y and the direction Z and a measurement position of the ultrasonic probe 50 in the direction Y and the direction Z are adjusted. In this processing, the ultrasonic endoscope 20 is inserted into the body cavity P1 to output an ultrasonic wave from the internal acoustic-electric converting portion 22. Then, with the ultrasonic probe 50 contacting a surface of the test object P, the ultrasonic wave output from the ultrasonic endoscope 20 is detected using the ultrasonic probe 50 while the ultrasonic probe 50 is moved in the direction Y and the direction Z. If the position of the ultrasonic probe 50 in the direction Y and the direction Z agrees with the position of the ultrasonic endoscope 20 so the ultrasonic probe 50 is located directly above the ultrasonic endoscope 20 as viewed in the direction -X, a maximum detection intensity is acquired for the ultrasonic probe 50. Thus, the position in the direction Y and the direction Z that produces the maximum intensity in detecting the ultrasonic wave is determined as a measurement position of the ultrasonic probe 50.

Next, the position of an internal detection tomographic image acquired by using the ultrasonic endoscope 20 and that of an external detection tomographic image acquired by using the ultrasonic probe 50 are adjusted. This processing is to achieve agreement between a plane on which the ultrasonic vibrating elements 22A belonging to the internal acoustic-electric converting portion 22 (see the B-B cross section of FIG. 4, for example) and a plane including a direction of arrangement of the ultrasonic vibrating elements 505A belonging to the internal acoustic-electric converting portion 505 (direction Z) and the direction X. First, the ultrasonic probe 50 detects the ultrasonic wave output from the ultrasonic endoscope 20 while being rotated about the direction X as an axis. If the aforementioned planes come into agreement, a maximum detection intensity is acquired for the ultrasonic probe 50. Thus, a rotation position that produces the maximum intensity in detecting the ultrasonic wave is determined as a measurement position of the ultrasonic probe 50.

Next, photoacoustic imaging is performed and a photoacoustic image is formed. The ultrasonic endoscope 20 and the ultrasonic probe 50 emit pulsed light to conform to the same timing of light emission. Then, an internal detection signal based on the photoacoustic wave AW detected by the ultrasonic endoscope 20 and an external detection signal based on the photoacoustic wave AW detected by the ultrasonic probe 50 are acquired. Next, based on an internal detection tomographic image generated from the internal detection signal and an external detection tomographic image generated from the external detection signal, a photoacoustic image is formed.

In the aforementioned photoacoustic imaging processing, the ultrasonic endoscope 20 and the ultrasonic probe 50 may emit pulsed light of the same PRF or different PRFs. Specifically, the ultrasonic endoscope 20 and the ultrasonic probe 50 are only required to emit light to conform to the same timing of light emission. The PRF of pulsed light from the ultrasonic endoscope 20 may be 1000 [times/sec] and the PRF of pulsed light from the ultrasonic probe 50 may be 500 [times/sec], for example.

### [Seventh Embodiment]

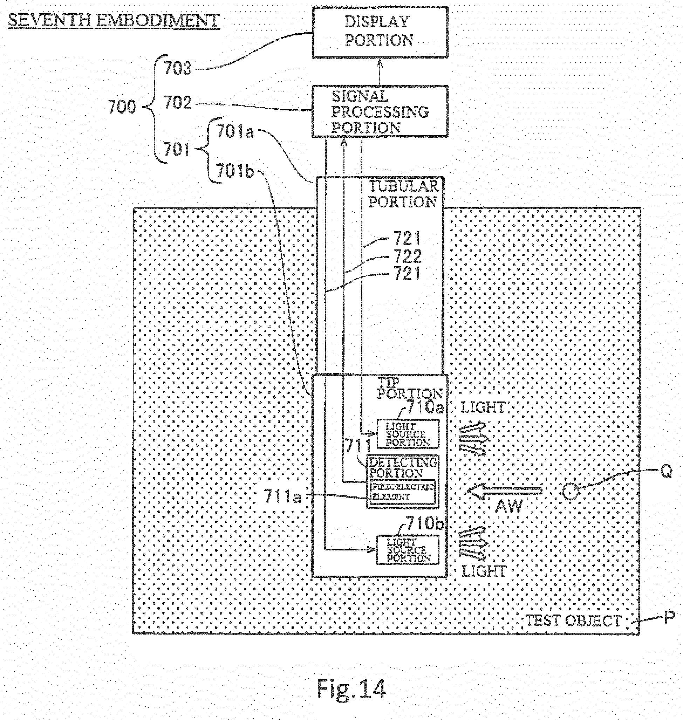

The configuration of a photoacoustic imaging apparatus 700 according to a seventh embodiment of the present invention will be described next by referring to FIGS. 14 to 18. According to an example described in the seventh embodiment, light from a light source is reflected on a reflective surface of a reflective portion toward a direction of a detection target facing a detection surface of a detecting portion.

As shown in FIG. 14, the photoacoustic imaging apparatus 700 according to the seventh embodiment of the present invention includes an endoscope body 701, a signal processing portion 702, and a display portion 703.

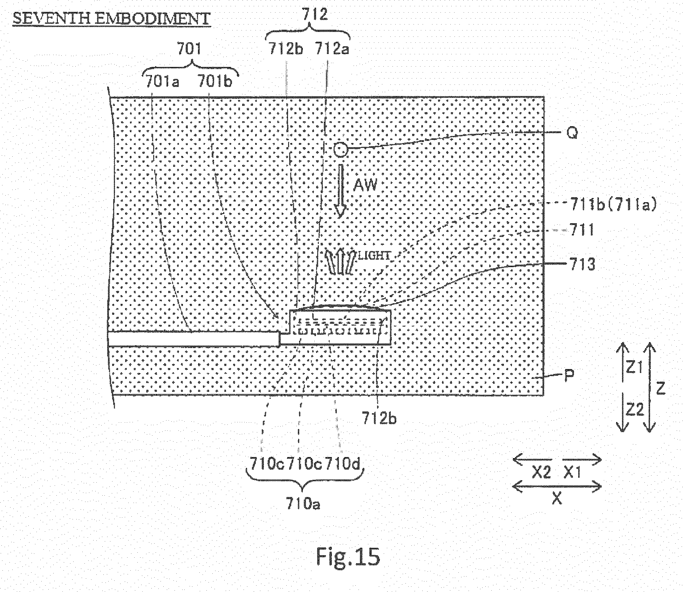

As shown in FIG. 15, the endoscope body 701 includes a tubular portion 701a and a tip portion 701 b provided at the tip of the tubular portion 701a. The endoscope body 701 is configured to be inserted into the test object (human body) P from the tip portion 701 b. The endoscope body 701 is an example of a "photoacoustic wave detector" according to the scope of claims.

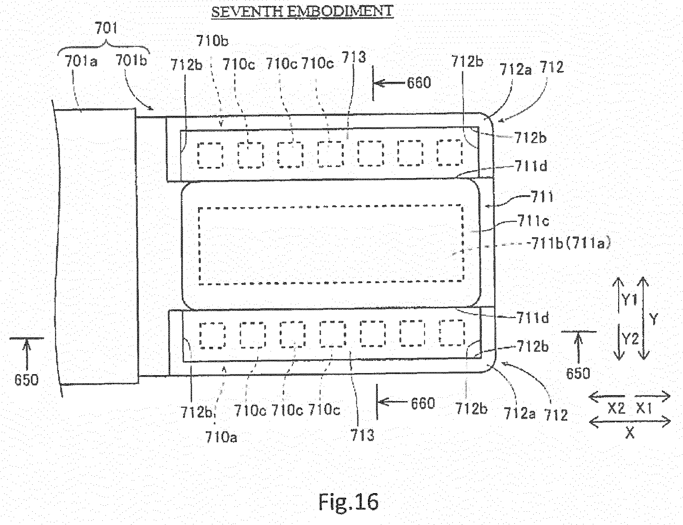

As shown in FIG. 16, in the seventh embodiment, the tip portion 701b of the endoscope body 701 includes an internal light source 710a and an internal light source 710b each including a plurality of LED elements 710c, an internal detecting portion 711 for detecting the photoacoustic wave AW (see FIG. 15), and a reflective portion 712 including a light reflective surface 712b. The internal detecting portion 711 includes a piezoelectric element 711 a having a detection surface 711 b. The reflective portion 712 is configured to reflect light from the LED element 710c on the reflective surface 712b toward a direction (direction Z1) of the detection target Q (see FIG. 15) facing the detection surface 711b, as will be described in detail later. The LED element 710c is an example of a "light-emitting element" according to the scope of claims.

The photoacoustic imaging apparatus 700 is configured to emit light from the internal light sources 710a and 710b to the test object P such as a human body (see FIG. 15) and to make the internal detecting portion 711 detect the photoacoustic wave AW generated from the detection target Q in the test object P when the detection target Q absorbs the emitted light. The photoacoustic imaging apparatus 700 is configured to be capable of forming an image of the detection target Q based on the photoacoustic wave AW detected by the internal detecting portion 711. The photoacoustic imaging apparatus 700 is configured to emit an ultrasonic wave from the piezoelectric element 711a to the test object P and to make the internal detecting portion 711 (piezoelectric element 711a) detect the ultrasonic wave reflected on the detection target Q in the test object P. The photoacoustic imaging apparatus 700 is configured to be capable of forming an image of the detection target Q based on the reflected ultrasonic wave detected by the internal detecting portion 711.

An ultrasonic wave referred to in this description denotes a sound wave (elastic wave) having such a high frequency as not to cause a sensation of hearing of a person with normal hearing ability, which is a sound wave (elastic wave) of about 16000 Hz or more. In this description, for the convenience of explanation, an ultrasonic wave generated by absorption of light by the detection target Q in the test object P will be called the "acoustic wave AW," an ultrasonic wave generated by the internal detecting portion 711 (piezoelectric element 711 a) and reflected on the detection target Q in the test object P will be called an "ultrasonic wave," and the "acoustic wave AW" and the "ultrasonic wave" will be described distinctively.

The configuration of each structure of the photoacoustic imaging apparatus 700 will be described next in detail.

As shown in FIG. 14, the signal processing portion 702 is connected to each of the internal light sources 710a and 710b through a signal line 721 through which power and signals are transmitted. The signal processing portion 702 is connected to the internal detecting portion 711 through a signal line 722 through which power and signals are transmitted. The signal processing portion 702 includes a CPU (not shown in the drawings) and storage portions such as a ROM and a RAM (not shown in the drawings). The signal processing portion 702 is configured to process a signal corresponding to the photoacoustic wave AW or an ultrasonic wave detected by the internal detecting portion 711. The signal processing portion 702 is configured to identify the detection target Q and to form an image of the identified detection target Q based on the signal corresponding to the photoacoustic wave AW or the ultrasonic wave detected by the internal detecting portion 711.

The display portion 703 is configured to be capable of displaying a photoacoustic image of the detection target Q in the test object P and various screens (an operational screen and a notification screen, for example) based on control under the signal processing portion 702.

The tubular portion 701a of the endoscope body 701 is formed into an elongated tubular shape so as to allow insertion of the tubular portion 701a into the test object P such as a human body. The tubular portion 701a has flexibility. The tubular portion 701a includes an operational portion (not shown in the drawings) provided near a base end of the tubular portion 701a opposite the tip thereof with the tip portion 701b. The operational portion is used for operating the tubular portion 701a and the tip portion 701 b during insertion of the endoscope body 701. The base end of the tubular portion 701a is not to be inserted into the test object P.

As described above, as shown in FIG. 16, the tip portion 701b of the endoscope body 701 includes the internal light sources 710a and 710b, the internal detecting portion 711, and the reflective portion 712.

The internal light sources 710a and 710b form a pair and are provided on opposite sides of the internal detecting portion 711 in a direction Y. Each of the internal light sources 710a and 710b is arranged near the internal detecting portion 711. The internal light sources 710a and 710b are configured to be substantially symmetric with respect to the internal detecting portion 711. Thus, only the internal light source 710a closer to a direction Y2 will be described below. A structure for reflecting and guiding light toward the test object P (see FIG. 15) corresponding to the internal light source 710a and a comparable structure corresponding to the internal light source 710b are also configured to be substantially symmetric with respect to the internal detecting portion 711. Thus, only the structure for reflection and guidance of light closer to the direction Y2 and corresponding to the internal light source 710a will be described below. A direction in which the tip portion 701 b and the tubular portion 701 a are aligned is defined as a lengthwise direction (direction X) of the tip portion 701b. A direction perpendicular to the detection surface 711b of the internal detecting portion 711 is defined as a thickness direction (direction Z) of the tip portion 701 b. A direction perpendicular to each of the lengthwise direction (direction X) and the thickness direction (direction Z) of the tip portion 701 b is defined as a width direction (direction Y) of the tip portion 701 b.

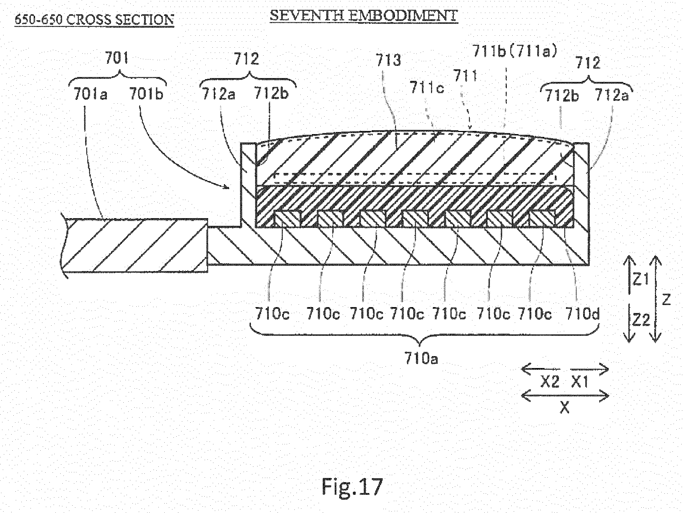

As shown in FIG. 14, the internal light source 710a is configured to generate the photoacoustic wave AW from the detection target Q in the test object P by emitting light to the test object P and making the detection target Q absorb the emitted light. As shown in FIG. 17, the internal light source 710a is formed to extend in the direction X.

The internal light source 710a includes the LED elements 710c and a sealing portion 710d that seals the LED elements 710c integrally. The tip portion 701b of the endoscope body 701 includes a light guide portion 713 that guides light from the internal light source 710a to the test object P (see FIG. 15).

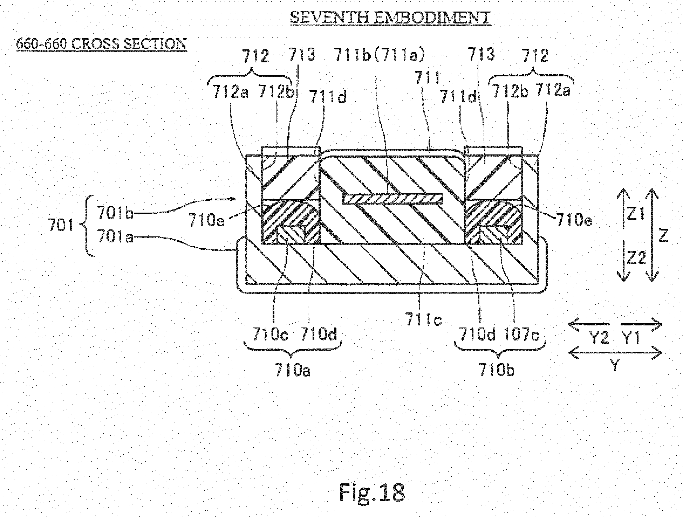

As shown in FIG. 18, the light guide portion 713 has a substantially rectangular cross section in a plane YZ. As shown in FIG. 17, the light guide portion 713 is formed into a rod-like shape extending in the direction X. The light guide portion 713 is arranged closer to the detection target (closer to the direction Z1) (see FIG. 15) than the sealing portion 710d. As understood from FIGS. 17 and 18, the light guide portion 713 is arranged to overlap the sealing portion 710d in a plan view (as viewed in the direction Z1).

The LED elements 710c are arranged in a line in the direction X to form a single LED element line. The LED elements 710c are configured to emit light toward the test object P within a range of a certain orientation angle with respect to the direction Z1.

The sealing portion 710d seals the LED elements 710c (single LED element line) integrally. As shown in FIG. 18, the sealing portion 710d has an outer surface 710e formed into a lens-like shape that collects light from the LED elements 710c and directs the collected light toward the light guide portion 713. More specifically, as viewed in the direction X, the sealing portion 710d has the outer surface 710e formed into a semi-arc-like shape having a projection pointed to the direction Z1. The outer surface 710e having the semi-arc-like shape extends in the direction X. The lower surface of the light guide portion 713 (a surface closer to a direction Z2) is formed as a flat surface extending in the directions X and Y.

The sealing portion 710d is made of a material having high light permeability. For example, the sealing portion 710d is made of silicone. An air layer is provided between the sealing portion 710d and the light guide portion 713. The sealing portion 710d has a different refractive index from the air layer (has a larger refractive index than the air layer). Thus, the sealing portion 710d is configured to make a direction of travel of light from the LED element 710c approach a direction (direction Z1) perpendicular to the lower surface of the light guide portion 713 by refracting the light on the outer surface 710e. Specifically, the sealing portion 710d is configured to refract light on the outer surface 710e so as to make the direction of travel of the light approach a direction (direction Z1) of the detection target Q (see FIG. 15) facing the detection surface 711b of the piezoelectric element 711a. The sealing portion 710d is in line contact with the lower surface of the light guide portion 713 (a surface closer to the direction Z2) at the top of the sealing portion 710d closer to the direction Z1.

The internal detecting portion 711 is formed to extend in the direction X while being caught between the internal light sources 710a and 710b in a pair arranged on opposite sides of the direction Y. The internal detecting portion 711 includes the piezoelectric element 711a having the detection surface 711b pointed to the direction Z1, and a resin portion 711c covering the piezoelectric element 711a.

The piezoelectric element 711a includes a large number of piezoelectric elements 711a arranged in an array pattern. The piezoelectric elements 711a are configured to form a rectangular shape as a whole extending in the direction X. Only the outer shape of the entire array pattern of the piezoelectric elements 711a is shown in outline in each of the drawings. As shown in FIG. 14, the piezoelectric element 711a is configured to be capable of detecting the photoacoustic wave AW and an ultrasonic wave and to be capable of emitting an ultrasonic wave to the test object P. The piezoelectric element 711a is configured to detect the photoacoustic wave AW generated from the detection target Q in the test object P and an ultrasonic wave reflected in the test object P, and to output a detection signal to the signal processing portion 702 through the signal line 722. The piezoelectric element 711a is configured to emit an ultrasonic wave to the test object P based on a control signal from the signal processing portion 702 transmitted to the piezoelectric element 711 a through the signal line 722.

As shown in FIG. 18, the resin portion 711c has a substantially rectangular cross section in the plane YZ and is formed to extend in the direction X. The resin portion 711c has a light reflective surface 711d forming an entire surface of the resin portion 711c adjacent to the internal light source 710a. The reflective surface 711d abuts on a surface of the internal light source 710a and a surface of the light guide portion 713 closer to a direction Y1.

As shown in FIG. 16, the reflective portion 712 includes a side wall 712a arranged lateral to the internal detecting portion 711 and the internal light source 710a, and the reflective surface 712b is provided on a surface of the side wall 712a adjacent to the internal detecting portion 711 and the internal light source 710a. More specifically, the reflective portion 712 has the side wall 712a formed into a U-shape extending in the direction X in a plan view (as viewed in the direction Z1) by being arranged to be adjacent to the LED elements 710c (LED element line) of the internal light source 710a in the direction X1, a direction X2, and the direction Y2. The reflective portion 712 has the reflective surface 712b abutting on a surface of the internal light source 710a and that of the light guide portion 713 closer to the direction X1, a surface of the internal light source 710a and that of the light guide portion 713 closer to the direction X2, and a surface of the internal light source 710a and that of the light guide portion 713 closer to the direction Y2.

In this way, the reflective surface 711d of the resin portion 711c and the reflective surface 712b of the reflective portion 712 are provided to surround the LED elements 710c of the internal light source 710a. Specifically, the LED elements 710c (LED element line) of the internal light source 710a are surrounded on sides (opposite sides of the direction X and opposite sides of the direction Y) by the reflective surface 712b of the reflective portion 712 and the reflective surface 711d of the internal detecting portion 711.

The seventh embodiment achieves the following effect.

As described above, in the seventh embodiment, the endoscope body 701 capable of being inserted into a test object includes the reflective portion 712 having the reflective surface 712b on which light generated from the LED element 710c of the endoscope body 701 is reflected toward a direction of a detection target facing the detection surface 711b. This allows the light generated from the LED element 710c of the endoscope body 701 to be emitted directly to the test object. This eliminates the need for a configuration conventionally required for guiding light from the internal light source 710a (710b) provided separately from the endoscope body 701 to the endoscope body 701, thereby contributing to simplification of the configuration of a light transmission path. Further, unlike in a conventional case of using a solid-state laser, using the LED element 710c can contribute to size reduction of the configuration of a light source. Further, as a result of provision of the reflective portion 712 having the reflective surface 712b to the endoscope body 701, emitted light not to travel in the direction of the detection target can be reflected on the reflective surface 712b toward the detection target. This allows efficient emission of light to the detection target.

As described above, in the seventh embodiment, the reflective portion 712 includes the side wall 712a arranged lateral to the internal detecting portion 711 and the internal light source 710a (710b), and the side wall 712a has the reflective surface 712b provided on a surface of the side wall 712a adjacent to the internal detecting portion 711 and the internal light source 710a (710b). Thus, even if a light-emitting diode likely to cause diffusion of light is used as the light-emitting element, for example, light can be emitted to a detection target in a well-balanced manner only by providing the reflective surface 712b as a simple structure. Specifically, well-balanced emission of light to the detection target is achieved without complicating the configuration of a light transmission path.

As described above, in the seventh embodiment, the light guide portion 713 that guides light from the internal light source 710a (710b) to a test object is provided. Further, the internal light source 710a (710b) includes the sealing portion 710d that seals the LED element 710c and the light guide portion 713 is arranged closer to a detection target than the sealing portion 710d. This makes it possible to guide light from the sealing portion 710d to the test object through the light guide portion 713, thereby suppressing loss of light energy occurring between the sealing portion 710d and the test object.

As described above, in the seventh embodiment, the sealing portion 710d has the outer surface 710e formed into a lens-like shape that collects light from the LED element 710c and directs the collected light toward the light guide portion 713. Thus, light from the LED element 710c can be collected and directed to the light guide portion 713, thereby suppressing loss of light energy such as failing to achieve arrival of light from the LED element 710c at the light guide portion 713.

As described above, in the seventh embodiment, the internal light source 710a (710b) includes the LED elements 710c and the reflective surfaces 711d and 712b are provided to surround the LED elements 710c. As a result of the presence of the reflective surfaces 711d and 712b surrounding the LED elements 710c, light emitted from the LED element 710c to be diffused to its surrounding can be reflected toward a detection target. As a result, the light can be emitted efficiently to the detection target.

### [Eighth Embodiment]

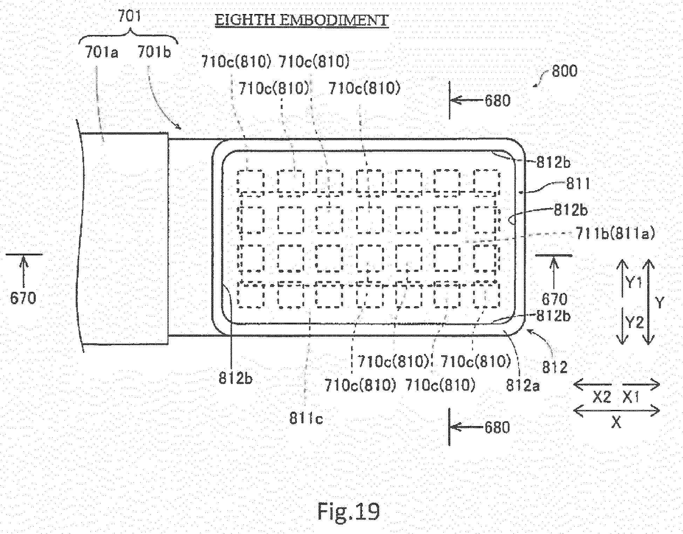

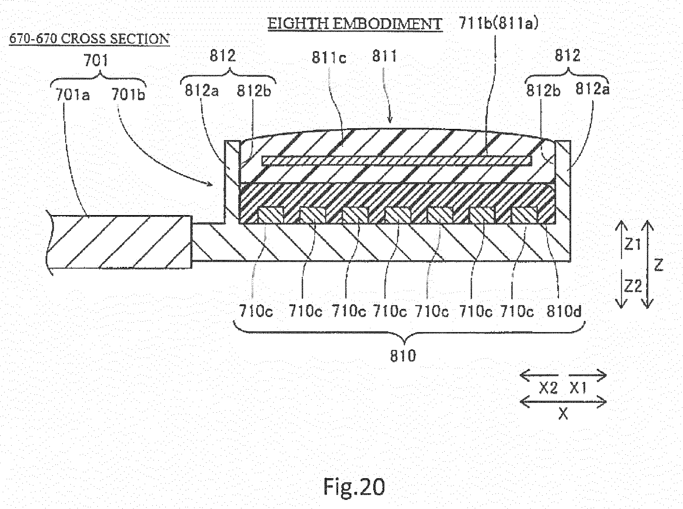

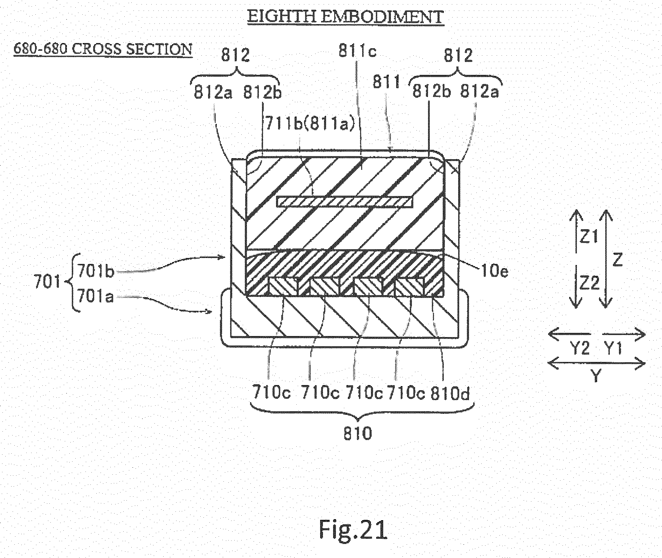

An eighth embodiment will be described next by referring to FIGS. 19 to 21. Unlike in the above-described seventh embodiment where the internal light sources 710a and 710b are arranged lateral to the internal detecting portion 711 (closer to the directions Y1 and Y2 than the internal detecting portion 711), in an example described below, an internal light source 810 is arranged in a direction (direction Z2) opposite a direction of a target of detection by an internal detecting portion 811. Structures similar to those of the above-described seventh embodiment will be given the same signs and description of such structures will be omitted.

As shown in FIGS. 19 to 21, in a photoacoustic imaging apparatus 800 according to the eighth embodiment, the internal detecting portion 811 includes a piezoelectric element 811a that allows transmission of light and a light guide resin portion 811 c. The piezoelectric element 811a is arranged in the light guide resin portion 811c and is arranged to overlap the LED element 710c in a plan view (as viewed in the direction Z1). The light guide resin portion 811c has the function of transmitting the photoacoustic wave AW and an ultrasonic wave and the function of guiding light from the LED element 710c to the test object P (see FIG. 14). The piezoelectric element 811a is made of a material having permeability and piezoelectricity. For example, the piezoelectric element 811a is made of PVDF (polyvinylidene fluoride). The light guide resin portion 811c is an example of a "light guide portion" according to the scope of claims.

The photoacoustic imaging apparatus 800 includes the internal light source 810 with the LED elements 710c and a sealing portion 810d that seals the LED elements 710c. The internal light source 810 is arranged below the piezoelectric element 811a (closer to the direction Z2 than the piezoelectric element 811 a). The LED elements 710c are arranged in an array pattern, with seven lines in the direction X and four lines in the direction Y. These LED elements 710c together form a surface light source.

The internal detecting portion 811 and the internal light source 810 are surrounded on sides (opposite sides of the direction X and opposite sides of the direction Y) by the reflective portion 812 including a side wall 812a and a reflective surface 812b. More specifically, the reflective portion 812 surrounds the entire periphery of the internal detecting portion 811 and that of the internal light source 810 arranged one above the other. In this way, the reflective surface 812b is formed to surround the entire periphery of the internal detecting portion 811 and that of the internal light source 810.

The eighth embodiment achieves the following effect.

Like in the seventh embodiment, in the eighth embodiment, the endoscope body 701 capable of being inserted into a test object includes the reflective portion 812 having the reflective surface 812b on which light generated from the LED element 710c of the endoscope body 701 is reflected toward a direction of a detection target facing the detection surface 711 b. This can contribute to simplification of the configuration of a light transmission path. This can further contribute to size reduction of the configuration of a light source.

As described above, in the eighth embodiment, the internal detecting portion 811 includes the piezoelectric element 811a that allows transmission of light. The piezoelectric element 811a is arranged in the light guide resin portion 811c and is arranged to overlap the LED element 710c in a plan view. Thus, the LED element 710c is not required to be arranged so as to bypass the position of the piezoelectric element 811a in a plan view. This can ensure a wider area for arrangement of the LED element 710c than arrangement where the LED element 710c and the piezoelectric element 811a do not overlap in a plan view. As a result, the quantity of light from the internal light source 810 can be increased.

As described above, in the eighth embodiment, the reflective surface 812b is provided in an annular shape to surround the entire peripheries of the LED elements 710c. This allows the reflective surface 812b to cause reflection of light over the entire peripheries of the LED elements 710c, so that the light can be emitted more efficiently to the detection target Q.

### [Ninth Embodiment]

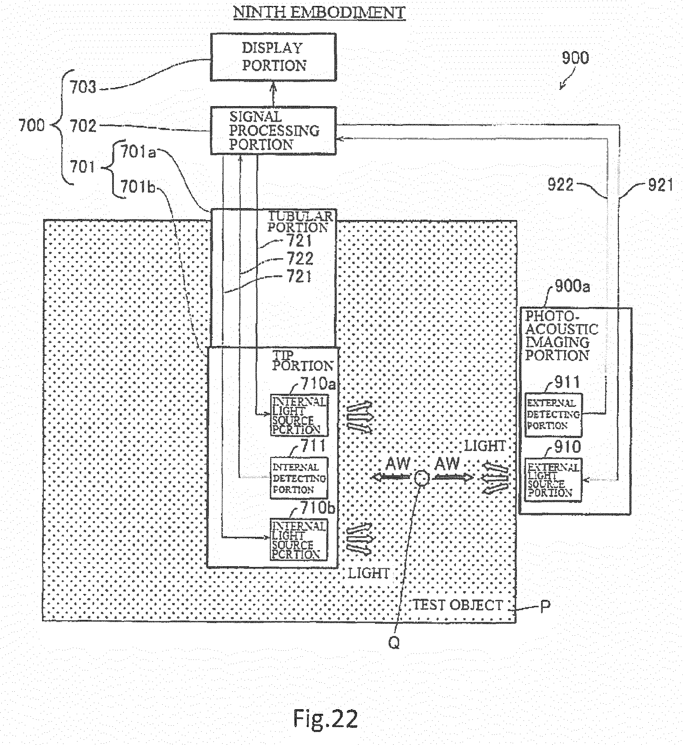

A ninth embodiment will be described next by referring to FIG. 22. In addition to the above-described configuration of the photoacoustic imaging apparatus 700 according to the seventh embodiment to emit light from the inside of the test object P and to detect the photoacoustic wave AW inside the test object P, an exemplary configuration described in the ninth embodiment includes a photoacoustic imaging portion 900a that emits light from the outside of the test object P and detects the photoacoustic wave AW outside the test object P. Structures similar to those of the seventh embodiment will be given the same signs and description of such structures will be omitted.

As shown in FIG. 22, a photoacoustic imaging apparatus 900 according to the ninth embodiment includes the photoacoustic imaging portion 900a arranged outside the test object P to form a photoacoustic endoscope system capable of emitting light from the inside and from the outside of the test object P and capable of detecting the photoacoustic wave AW inside and outside the test object P.

The photoacoustic imaging portion 900a includes an external light source 910 that emits light from the outside of the test object P, and an external detecting portion 911 for detecting the photoacoustic wave AW generated from the detection target Q in the test object P when the detection target Q absorbs light emitted from the external light source 910. The photoacoustic imaging portion 900a is configured to be capable of generating an ultrasonic wave from the external detecting portion 911.

The external light source 910 is connected to the signal processing portion 702 through a signal line 921. The external detecting portion 911 is connected to the signal processing portion 702 through a signal line 922.

The signal processing portion 702 is configured to bring timing of emission of internal light 810a (810b) to the test object P into synchronization with timing of emission of light from the external light source 910 to the test object P. Specifically, the internal light source 710a (710b) and the external light source 910 are configured to emit light to the test object P simultaneously for respective detections of the photoacoustic waves AW by the internal detecting portion 711 and the external detecting portion 911.

The signal processing portion 702 is configured to superimpose respective photoacoustic images based on the photoacoustic waves AW detected by the internal detecting portion 711 and the external detecting portion 911. The internal detecting portion 711 and the external detecting portion 911 are configured in such a manner that, before execution of processing of superimposing the photoacoustic images, an ultrasonic wave is generated from one of the internal light source 710a (710b) and the external light source 910 to adjust the positions of the internal detecting portion 711 and the external detecting portion 911 relative to each other.

The ninth embodiment achieves the following effect.

Like in the seventh embodiment, in the ninth embodiment, the endoscope body 701 capable of being inserted into a test object includes the reflective portion 712 having the reflective surface 712b on which light generated from the LED element 710c of the endoscope body 701 is reflected toward a direction of a detection target facing the detection surface 711b. This can contribute to simplification of the configuration of a light transmission path. This can further contribute to size reduction of the configuration of a light source.

As described above, in the ninth embodiment, the photoacoustic endoscope 900 is provided that includes: the internal light source 710a (710b) provided to the endoscope body 701 capable of being inserted into a test object and including the LED element 710c that emits light from the inside of the test object; the internal detecting portion 711 provided to the endoscope body 701 and having the detection surface 711 b for detecting the photoacoustic wave AW generated from a detection target in the test object when the detection target absorbs light emitted from the internal light source 710a (710b); and the reflective portion 712 provided to the endoscope body 701 and functioning to reflect light from the LED element 710c toward a direction of the detection target facing the detection surface 711 b. Further, the photoacoustic imaging portion 300a is provided that includes: the external light source 910 that emits light from the outside of the test object; and the external detecting portion 911 for detecting the photoacoustic wave AW generated from the detection target in the test object when the detection target absorbs light emitted from the external light source 910. In this way, the external light source 910 becomes usable as a light source auxiliary to the internal light source 710a (710b), so that light of a high intensity can be emitted to the detection target, compared to emission of light only from the internal light source 710a (710b). As a result, compared to emission of light only from the internal light source 710a (710b), the larger photoacoustic wave AW can be generated, so that a resultant photoacoustic image can be given a higher degree of clearness. Additionally, photoacoustic images are acquired both from the internal detecting portion 711 inside the test object and the external detecting portion 911. Thus, a resultant photoacoustic image is allowed to cover a wider range of the test object.

As described above, in the ninth embodiment, the signal processing portion 702 is provided and the signal processing portion 702 is configured to superimpose photoacoustic images based on corresponding photoacoustic waves AW detected by the internal detecting portion 711 and the external detecting portion 911 respectively. By doing so, photoacoustic images acquired from corresponding respective photoacoustic waves AW detected by two different detecting portions respectively are superimposed. This makes it possible to reduce a noise component in a detection signal relatively to a signal component in the detection signal forming a photoacoustic image. As a result, a resultant photoacoustic image can be given a still higher degree of clearness

### [Modifications]

The embodiments disclosed herein must be considered to be illustrative in all aspects and not restrictive. The range of the present invention is understood not by the above description of the embodiments but by the scope of claims for patent. All changes (modifications) within the meaning and range equivalent to the scope of claims for patent are to be embraced.

For example, in the example shown in above-described seventh to ninth embodiments, the reflective surface is provided to surround the LED element. However, this is not to limit the present invention. In the present invention, the reflective surface may be provided in such a manner that the LED element is not surrounded but is exposed partially in a certain direction.

In the example shown in above-described seventh to ninth embodiments, the internal light source is configured to include LED elements arranged in a line. However, this is not to limit the present invention. In the present invention, the internal light source may be configured to include LED elements arranged in a plurality of lines.

In the example shown in above-described seventh to ninth embodiments, the light guide portion or the light guide resin portion and the sealing portion are arranged to contact each other. However, this is not to limit the present invention. In the present invention, the light guide portion or the light guide resin portion and the sealing portion may also be arranged to be separated from each other.

### Reference Sings List

20 Photoacoustic endoscope (photoacoustic wave detector)
20a Inserted part
22 Internal acoustic-electric converting portion (internal detecting portion)
212, 710a, 710b, 810 Internal light source
212a, 710c LED element (light-emitting element)
214 Reflective portion (reflective member)
214a, 215a, 712b, 812b Reflective surface
215 Reflective plate (reflective member)
504A, 504B, 910 External light source
505 External acoustic-electric converting portion (external detecting portion)
701 Endoscope body (photoacoustic wave detector)
702 Signal processing portion
710d, 810d Sealing portion
710e Outer surface
711, 811 Internal detecting portion
711 b Detection surface
712, 812 Reflective portion
712a, 812a Side wall
713 Light guide portion
700, 800, 900 Photoacoustic imaging apparatus
811a Piezoelectric element
811c Light guide resin portion (light guide portion)
900a Photoacoustic imaging portion
911 External detecting portion
P Test object
Q Detection target
AW Photoacoustic wave

## Claims

1. A photoacoustic wave detector comprising:
an internal light source (212, 710a, 710b, 810) including a light-emitting element (212a, 710c) to be inserted into a test object (150, P), the internal light source applying light emitted from the light-emitting element to a detection target (Q) in the test object; and
an internal detecting portion (22, 711, 811) that detects a photoacoustic wave inside the test object and generates an internal detection signal based on a result of detection of the photoacoustic wave, the photoacoustic wave being generated at the detection target.

2. The photoacoustic wave detector according to claim 1, wherein
the internal light source and the internal detecting portion are both provided near the tip of an inserted part (20a) to be inserted into the test object.

3. The photoacoustic wave detector according to claim 1, wherein
the internal detecting portion is provided at a lateral surface of the inserted part (20a) to be inserted into the test object, the lateral surface extending in a direction conforming to a direction of insertion of the inserted part,
the lateral surface is formed of a curved surface or a plurality of planes and includes a mounting surface on which the light-emitting element is mounted, and
the light-emitting element is arranged to be pointed outward of a direction of a normal to the mounting surface of the inserted part.

4. The photoacoustic wave detector according to claim 3, wherein
the mounting surface is formed of a circular curved surface,
the internal detecting portion is provided along the outer periphery of the lateral surface of the inserted part as viewed in the direction of insertion of the inserted part, and
the light-emitting element is configured to emit light outward of the direction of the normal so as to cover the entire periphery of the mounting surface as viewed in the direction of insertion of the inserted part.

5. The photoacoustic wave detector according to claim 3, further comprising a reflective member (214, 215) that reflects light emitted from the light-emitting element outward of the direction of the normal.

6. The photoacoustic wave detector according to claim 1, further comprising a reflective member (214) that reflects light emitted from the light-emitting element outward of a direction of a normal to a lateral surface of the inserted part (20a) to be inserted into the test object, the lateral surface extending in a direction conforming to a direction of insertion of the inserted part, wherein
the internal detecting portion is provided at the lateral surface,
the light-emitting element is mounted on a mounting surface pointed in the direction of insertion, and
the reflective member has a reflective surface (214a) facing the mounting surface.

7. The photoacoustic wave detector according to claim 1, wherein
the inserted part (20a) to be inserted into the test object is rotatable about the direction of insertion of the inserted part as an axis.

8. The photoacoustic wave detector according to claim 1, wherein
the internal light source includes at least one of a light-emitting diode element, a semiconductor laser element, and an organic light-emitting diode element to function as the light-emitting element.

9. The photoacoustic wave detector according to claim 1, wherein
the internal detecting portion has a detection surface (711 b) provided near the internal light source and used for detecting a photoacoustic wave,
the photoacoustic wave detector further comprising a reflective portion (712, 812) having a reflective surface (712b, 812b) on which light generated from the light-emitting element is reflected toward a direction of the detection target facing the detection surface.

10. The photoacoustic wave detector according to claim 9, wherein
the reflective portion includes a side wall (712a, 812a) arranged lateral to the internal detecting portion and the internal light source, and
the reflective surface is provided on a surface of the side wall adjacent to the internal detecting portion and the internal light source.

11. The Photoacoustic wave detector according to claim 9, further comprising a light guide portion (713, 811c) that guides light from the internal light source to the test object, wherein
the internal light source includes a sealing portion (710d, 810d) that seals the light-emitting element, and
the light guide portion is arranged closer to the detection target than the sealing portion.

12. The photoacoustic wave detector according to claim 11, wherein
the sealing portion has an outer surface (710e) formed into a lens-like shape that collects light from the light-emitting element and directs the collected light toward the light guide portion.

13. The photoacoustic wave detector according to claim 11, wherein
the internal detecting portion includes a piezoelectric element (811a) that allows transmission of light, and
the piezoelectric element is arranged in the light guide portion and is arranged to overlap the light-emitting element in a plan view.

14. The photoacoustic wave detector according to claim 9, wherein
the internal light source includes a plurality of the light-emitting elements, and
the reflective surface is provided to surround the light-emitting elements.

15. The photoacoustic wave detector according to claim 14, wherein
the reflective surface is provided in an annular shape to surround the entire peripheries of the light-emitting elements.

16. A photoacoustic imaging apparatus comprising:
a photoacoustic wave detector including an internal light source (212, 710a, 710b, 810) and an internal detecting portion (22, 711, 811), the internal light source including a light-emitting element (212a, 710c) to be inserted into a test object, the internal light source applying light emitted from the light-emitting element to a detection target in the test object, the internal detecting portion detecting a photoacoustic wave inside the test object and generating an internal detection signal based on a result of detection of the photoacoustic wave, the photoacoustic wave being generated at the detection target;
an internal detection image generating portion that generates an internal detection tomographic image of the test object based on the internal detection signal; and
an image forming portion that forms a photoacoustic image using the internal detection tomographic image.

17. The photoacoustic imaging apparatus according to claim 16, further comprising an external light source (504A, 504B, 910) that emits light from the outside of the test object to the detection target, wherein
timing of emission of light from the external light source is synchronized with timing of emission of light from the internal light source.

18. The photoacoustic imaging apparatus according to claim 16, further comprising:
an external detecting portion (505, 911) that detects a photoacoustic wave outside the test object and generates an external detection signal based on a result of detection of the photoacoustic wave, the photoacoustic wave being generated at the detection target in the test object; and
an external detection image generating portion that generates an external detection tomographic image of the test object based on the external detection signal, wherein
the image forming portion further uses the external detection tomographic image for forming the photoacoustic image.

19. The photoacoustic imaging apparatus according to claim 18, wherein
the internal detecting portion has a detection surface (711b) provided near the internal light source and used for detecting a photoacoustic wave, and
the photoacoustic wave detector further includes a reflective portion (712, 812) having a reflective surface (712b, 812b) on which light generated from the light-emitting element is reflected toward a direction of the detection target facing the detection surface.

20. The photoacoustic imaging apparatus according to claim 19, further comprising a signal processing portion (702), wherein
the signal processing portion is configured to superimpose photoacoustic images based on corresponding photoacoustic waves detected by the internal detecting portion and the external detecting portion respectively.
